# EUROPEAN PATENT SPECIFICATION

(11) **EP 1 423 429 B1**
(45) Date of publication and mention of the grant of the patent: **09.08.2006**
(21) Application number: 02762749.6
(22) Date of filing: 04.09.2002
(51) Int. Cl.: C07K 16/40

(54) **Antibodies against caspase-8, their preparation and use**
Antikörper gegen Caspase-8, ihre Herstellung und Verwendung
Anticorps anti-caspase-8, preparation et utilisation de ces anticorps

(30) Priority: 04.09.2001 IL 14527901
(43) Date of publication of application: 02.06.2004
(73) Proprietor: YEDA RESEARCH AND DEVELOPMENT CO., LTD., 76100 Rehovot (IL)
(72) Inventor: WALLACH, David, 76100 Rehovot (IL); GONCHAROV, Tanya, 76281 Rehovot (IL); KOLUMAM, Ganesh, Seattle, WA 98105 (US)
(74) Representative: Weiss, Wolfgang
(86) International application number: PCT/IL2002/000734
(87) International publication number: WO 2003/020767

(56) References cited:
- WO-A-00/77201
- MEDEMA JAN PAUL ET AL: "Bcl-xL acts downstream of caspase-8 activation by the CD95 death-inducing signaling complex." JOURNAL OF BIOLOGICAL CHEMISTRY, vol. 273, no. 6, 6 February 1998 (1998-02-06), pages 3388-3393, XP002229994 ISSN: 0021-9258
- "Anti-caspase 8 (GD-13); Product number C2976" SIGMA PRODUCT INFORMATION, [Online] September 1999 (1999-09), XP002229995 Retrieved from the Internet: <URL:http://www.sigmaaldrich.com/ProductLo okup.html?ProdNo=C2976&Brand=SIGMA> [retrieved on 2003-01-24]
- "Caspase-8 (1C12) Monoclonal Antibody" CELL SIGNALING PRODUCT SPECIFICATION, [Online] 1999, XP002229996 Retrieved from the Internet: <URL:http://www.cellsignal.com/product.asp ?catalog%5Fname=CellSignal&category%5Fname =Apoptosis+%2F+Caspase&product%5Fid=9746> [retrieved on 2003-01-24]
- RODIER F ET AL: "Polyomavirus large T-antigen protects mouse cells from Fas-, TNF-alpha- and taxol-induced apoptosis." ONCOGENE, vol. 19, no. 54, 2000, pages 6261-6270, XP008012999 ISSN: 0950-9232
- BOLDIN M P ET AL: "INVOLVEMENT OF MACH, A NOVEL MORT1/FADD-INTERACTING PROTEASE, IN FAS/APO-1- AND TNF RECEPTOR-INDUCED CELL DEATH" CELL, CELL PRESS, CAMBRIDGE, NA, US, vol. 85, no. 6, 14 June 1996 (1996-06-14), pages 803-815, XP002037501 ISSN: 0092-8674
- NIIKURA YUICHI ET AL: "Monitoring of caspase-8/FLICE processing and activation upon Fas stimulation with novel antibodies directed against a cleavage site for caspase-8 and its substrate, FLICE-like inhibitory protein (FLIP)." JOURNAL OF BIOCHEMISTRY (TOKYO), vol. 132, no. 1, July 2002 (2002-07), pages 53-62, XP001109528 ISSN: 0021-924X

## Description

### FIELD OF THE INVENTION

The invention relates to antibodies to a specific region in caspase-8, and to their use.

### BACKGROUND OF THE INVENTION

Tumor Necrosis Factor (TNF-alpha) and Lymphotoxin (TNF-beta) are multifunctional proinflammatory cytokines formed mainly by mononuclear leukocytes, which have many effects on cells (Wallach, D. (1986) In: Interferon 7 (Ion Gresser, ed.), pp. 83-122, Academic Press, London; and Beutler and Cerami (1987). Both TNF-alpha and TNF-beta initiate their effects by binding to specific cell surface receptors. Some of the effects are likely to be beneficial to the organism: they may destroy, for example, tumor cells or virus infected cells and augment antibacterial activities of granulocytes. In this way, TNF contributes to the defense of the organism against tumors and infectious agents and contributes to the recovery from injury. Thus, TNF can be used as an anti-tumor agent in which application it binds to its receptors on the surface of tumor cells and thereby initiates the events leading to the death of the tumor cells. TNF can also be used as an anti-infectious agent.

However TNF-alpha has deleterious effects. There is evidence that overproduction of TNF-alpha may play a major pathogenic role in several diseases. For example, effects of TNF-alpha, primarily on the vasculature, are known to be a major cause for symptoms of septic shock (Tracey et al, 1994). In some diseases, TNF may cause excessive loss of weight (cachexia) by suppressing activities of adipocytes and by causing anorexia, and TNF-alpha was thus called cachectin. It was also described as a mediator of the damage to tissues in rheumatic diseases (Beutler and Cerami, 1987) and as a major mediator of the damage observed in graft-versus-host reactions (Grau GE et al.,1989). In addition, TNF is known to be involved in the process of inflammation and in many other diseases.

Two distinct, independently expressed receptors, the p55 (CD120a) and the p75 (CD120b) TNF-receptors, which bind both TNF-alpha and TNF-beta specifically, initiate and/or mediate the above noted biological effects of TNF. These two receptors have structurally dissimilar intracellular domains suggesting that they signal differently (See Hohmann et al., 1989; Engelmann et al., 1990; Brockhaus et al., 1990; Loetscher et al., 1990; Schall et al., 1990; Nophar et al., 1990; Smith et al., 1990). However, the cellular mechanisms, for example, the various proteins and possibly other factors, which are involved in the intracellular signaling of the CD120a and CD120b, have yet to be elucidated. It is intracellular signaling, which occurs usually after the binding of the ligand, i.e., TNF (alpha or beta), to the receptor that is responsible for the commencement of the cascade of reactions that ultimately result in the observed response of the cell to TNF.

As regards the above-mentioned cytocidal effect of TNF, in most cells studied so far, this effect is triggered mainly by CD120a. Antibodies against the extracellular domain (ligand binding domain) of CD120a can themselves trigger the cytocidal effect (see EP 412486) which correlates with the effectiveness of receptor cross-linking by the antibodies, believed to be the first step in the generation of the intracellular signaling process. Further, mutational studies (Brakebusch et al., 1992; Tartaglia et al, 1993) have shown that the biological function of CD120a depends on the integrity of its intracellular domain, and accordingly it has been suggested that the initiation of intracellular signaling leading to the cytocidal effect of TNF occurs as a consequence of the association of two or more intracellular domains of CD120a. Moreover, TNF (alpha and beta) occurs as a homotrimer, and as such, has been suggested to induce intracellular signaling via CD120a by way of its ability to bind to and to cross-link the receptor molecules, i.e., cause receptor aggregation (Engelmann H. et al 1990).

Another member of the TNF/NGF superfamily of receptors is the FAS/AP01 receptor (CD95). CD95 mediates cell death in the form of apoptosis (Itoh et al., 1991), and appears to serve as a negative selector of autoreactive T cells, i.e., during maturation of T cells, CD95 mediates the apoptotic death of T cells recognizing self-antigens. It has also been found that mutations in the CD95 gene (1pr) cause a lymphoproliferation disorder in mice that resembles the human autoimmune disease systemic lupus erythematosus (SLE) (Watanabe-Fukunaga et al., 1992). The ligand for CD95 is a cell-surface associated molecule carried by, amongst others, killer T cells (or cytotoxic T lymphocytes - CTLs), and hence when such CTLs contact cells carrying CD95, they are capable of inducing apoptotic cell death of the CD95-carrying cells. Further, monoclonal antibodies have been prepared that are specific for CD95, these monoclonal antibody being capable of inducing apoptotic cell death in cells carrying CD95, including mouse cells transformed by cDNA encoding human CD95 (e.g. Itoh et al., 1991).

TNF receptor and Fas signaling mechanisms comprising the different receptors, their regulation, and the down stream signaling molecules identified are reviewed in detailed by Wallach et al (1999).

It has been found that certain malignant cells and HIV-infected cells carry CD95 on their surface, antibodies against CD95, or the CD95 ligand, may be used to trigger the CD95 mediated cytotoxic effects in these cells and thereby provide a means for combating such malignant cells or HIV-infected cells (see Itoh et al., 1991). Finding yet other ways for enhancing the cytotoxic activity of CD95 may therefore also have therapeutic potential.

It has been a long felt need to provide a way for modulating the cellular response to TNF (alpha or beta) and CD95 ligand. For example, in the pathological situations mentioned above, where TNF or CD95 ligand is overexpressed, it is desirable to inhibit the TNF- or CD95 ligand-induced cytocidal effects, while in other situations, e.g., wound healing applications, it is desirable to enhance the TNF effect, or in the case of CD95, in tumor cells or HIV-infected cells, it is desirable to enhance the CD95 mediated effect.

A number of approaches have been made by the applicants (see, for example, European patent specifications Nos. EP 186,833. EP 308,378, EP 398,327 and EP 412,486) to regulate the deleterious effects of TNF by inhibiting the binding of TNF to its receptors using anti-TNF antibodies or by using soluble TNF receptors (being essentially the soluble extracellular domains of the receptors) to compete with the binding of TNF to the cell surface-bound TNF-receptors (TNF-Rs). Further, on the basis that TNF-binding to its receptors is required for the TNF-induced cellular effects, approaches by applicants (see for example EP 568,925) have been made to modulate the TNF effect by modulating the activity of the TNF-Rs.

EP 568,925 relates to a method of modulating signal transduction and/or cleavage in TNF-Rs whereby peptides or other molecules may interact either with the receptor itself or with effector proteins interacting with the receptor, thus modulating the normal function of the TNF-Rs. In EP 568,925, there is described the construction and characterization of various mutant forms of CD120a, having mutations in its extracellular, transmembrane and intracellular domains. In this way, regions within the above domains of CD120a were identified as being essential to the functioning of the receptor, i.e., the binding of the ligand (TNF) and the subsequent signal transduction and intracellular signaling which ultimately results in the observed TNF-effect on the cells. Further, there are also described a number of approaches to isolate and identify proteins, peptides or other factors which are capable of binding to the various regions in the above domains of CD120a, which proteins, peptides and other factors may be involved in regulating or modulating the activity of TNF-Rs. A number of approaches for isolating and cloning the DNA sequences encoding such proteins and peptides; for constructing expression vectors for the production of these proteins and peptides; and for the preparation of antibodies or fragments thereof which interact with CD120a or with the above proteins and peptides that bind various regions of CD120a are also set forth in EPO 368,925. However, EP 568,925 does not specify the actual proteins and peptides which bind to the intracellular domains of the TNF-Rs. Similarly, in EP 568,925 there is no disclosure of specific proteins or peptides capable of binding the intracellular domain of CD95.

Thus, when it is desired to inhibit the effect of TNF, or of the CD95 ligand, it would be desirable to decrease the amount or the activity of TNF-Rs or CD95 at the cell surface, while an increase in the amount or the activity of TNF-R or CD95 would be desired when an enhanced TNF or CD95 ligand effect is sought. To this end the promoters of both the CD120a, and the CD120b have been sequenced, analyzed and a number of key sequence motifs have been found that are specific to various transcription regulating factors, and as such the expression of these TNF-Rs can be controlled at their promoter level, i.e., inhibition of transcription from the promoters for a decrease in the number of receptors, and an enhancement of transcription from the promoters for an increase in the number of receptors (EP 606,869 and WO 9531206).

While it is known that the tumor necrosis factor (TNF) receptors, and the structurally related receptor CD95, trigger in cells, upon stimulation by leukocyte-produced ligands, destructive activities that lead to their own demise, the mechanisms of this triggering are still little understood. Mutational studies indicate that in CD95 and CD120a signaling for cytotoxicity involve distinct regions within their intracellular domains (Brakebusch et al., 1992; Tartaglia et al., 1993. Itoh and Nagata, 1993). These regions (the 'death domains) have sequence similarity. The 'death domains' of both CD95 and CD120a tend to self-associate. Their self-association apparently promotes the receptor aggregation, which is necessary for initiation of signaling (see Bigda et al.,1994; Boldin et al., 1995), and at high levels of receptor expression can result in triggering of ligand-independent signaling (Boldin et al.,1995).

Some of the cytotoxic effects of lymphocytes are mediated by interaction of a lymphocyte-produced ligand with CD95 of the target cell (see also Nagata and Goldstein, 1995). Cell killing by mononuclear phagocytes involves TNF and its receptor CD120a (see also Vandenabeele et al. 1995). Like other receptor-induced effects, cell death induction by the TNF receptors and CD95 occurs via a series of protein-protein interactions, leading from ligand-receptor binding to the eventual activation of enzymatic effector functions, which have been shown to comprise non-enzymatic protein-protein interactions that initiate signaling for cell death: binding of trimeric TNF or the CD95 ligand molecules to the receptors, the resulting interactions of their intracellular domains (Brakebusch et al., 1992; Tartaglia et al., 1993; Itoh and Nagata, 1993) augmented by a propensity of the death-domain motifs to self-associate (Boldin et al., 1995a), and induced binding of two cytoplasmic proteins (which can also bind to each other) to the receptors' intracellular domains - MORT-1 (or FADD) to CD95 (Boldin et al., 1995b; Chinnaiyan et al., 1995; Kischkel et al., 1995) and TRADD to CD120a (Hsu et al., 1996). Besides their binding to CD95 and CD 120a, MORT-1 and TRADD are also capable of binding to each other, as well as to other death domain containing proteins, such as RIP (Stanger et al. 1995), which provides for a functional "cross-talk" between CD95 and CD120a. These bindings occur through a conserved sequence motif, the 'death domain module' common to the receptors and their associated proteins. Furthermore, although in the yeast two-hybrid test MORT-1 was shown to bind spontaneously to CD95, in mammalian cells, this binding takes place only after stimulation of the receptor, suggesting that MORT-1 participates in the initiating events of CD95 signaling. MORT-1 does not contain any sequence motif characteristic of enzymatic activity, and therefore, its ability to trigger cell death seems not to involve an intrinsic activity of MORT-1 itself, but rather, activation of some other protein(s) that bind MORT-1 and act further downstream in the signaling cascade. Cellular expression of MORT-1 mutants lacking the N-terminal part of the molecule have been shown to block cytotoxicity induction by CD95 or CD120a (Hsu et al., 1996; Chinnaiyan et al., 1996), indicating that this N-terminal region transmits the signaling for the cytocidal effect of both receptors through protein-protein interactions.

A group of cytoplasmic thiol proteases, which are structurally related to the *Caenorhabditis elegans* protease CED3 and to the mammalian interleukin-1 beta-converting enzyme (ICE) have been implicated in the onset of various physiological cell death processes (reviewed in Kumar, 1995 and Henkart, 1996). There have also been evidences that protease(s) of this family take part in the cell-cytotoxicity induced by CD95 and TNF-Rs. Specific peptide inhibitors of the proteases and two virus-encoded proteins that block their function, the cowpox protein crmA and the Baculovirus p35 protein, were found to provide protection to cells against this cell-cytotoxicity (Enari et al., 1995; Tewari et al., 1995; Xue et al., 1995; Beidler et al., 1995). Rapid cleavage of certain specific cellular proteins, apparently mediated by protease(s) of the CED3/ICE (caspase) family, could be demonstrated in cells shortly after stimulation of CD95 or TNF-Rs.

One such protease and various isoforms thereof (including inhibitory ones), is known as MACH (now caspase-8) which is a MORT-1 binding protein has been isolated, cloned, characterized, and its possible uses also described, as is set forth in detail and incorporated herein in their entirety by reference, in co-owned PCT/US96/10521, and in a publication of the present inventors (Boldin et al., 1996). Another such protease and various isoforms thereof (including inhibitory ones), designated Mch4 (also called caspase-10) has also been isolated and characterized by the present inventors (unpublished) and others (Femandes-Alnemri et al., 1996; Srinivasula et al., 1996). Caspase-10 is also a MORT-1 binding protein. Thus, details concerning all aspects, features, characteristics and uses of caspase-10 are set forth in the above noted publications.

It should also be noted that the caspases, caspase-8 and caspase-10, which have similar pro-domains (see Boldin et al., 1996; Muzio et al., 1996; Fernandes-Alnemri et al., 1996; Vincent and Dixit, 1997) interact through their pro-domains with MORT-1, this interaction being via the 'death effector domain', DED, present in the N-terminal part of MORT-1 and present in duplicate in caspase-8 and caspase-10 (see Boldin et al., 1995b; Chinnalyan et al., 1995).

Caspases (cysteine aspartate-specific proteinases), are a growing family of cysteine proteases that share several common features. Most of the caspases have been found to participate in the initiation and execution of programmed cell death or apoptosis, while the others appear to be involved in the production of proinflammatory cytokines (Nicholson DW et al. 1997, Salvesen GS et al.1997, Cohen GM 1997). They are synthesized as catalytically almost inactive precursors and are generally activated by cleavage after specific internal aspartate residues present in interdomain linkers. The cleavage sites of caspases are defined by tetrapeptide sequences (X-X-X-D) and cleavage always occurs downstream of the aspartic acid. As a result certain mature active caspases can process and activate their own as well as other inactive precursors (Femandes-Alnemri T et al. 1996, Srinivasula SM et al.1996).

Activation of the programmed cell death process is generally specific and involves sequential processing of downstream caspases named "executioner" caspases by upstream caspases named "initiator" caspases. The functional characteristics of the two classes of caspases are also reflected by their structure. In fact the "initiator caspases" contain longer pro-domain regions as compared to the "executioner" caspases (Salvesen GS et al. 1997, Cohen GM 1997). The long pro-domain allows the initiator or "'apical" caspases to be activated by triggering of the death receptors of the TNF receptor family. Upon ligand-induced trimerization of the death receptors, the initiator caspases are recruited through their long N-terminal pro-domain to interact with specific adapter molecules to form the death inducing signaling complex (Cohen GM 1997, Kischkel FC et al., 1995). For example, caspase-8/MACH and probably caspase-10, which contain two DEDs, are recruited to the receptor complex by the adapter molecules FADD/MORT-1, whereas caspase-2 is assumed to be recruited by CRADD/RAIDD and RIP (Nagata S et al. 1997, MacFarlane M et al. 1997, Ahmad M et al. 1997, Duan H et al. 1997). Due to the trimeric nature of the activated receptor complex, at least two caspase molecules are thought to be brought in close proximity to each other, thus leading to their activation by auto-catalytic processing (Yang et al. 1998, Muzio et al. 1998).

Caspases are synthesized as pro-enzymes consisting of three major subunits, the N-terminal pro-domain, and two subunits, which are sometimes separated by a linker peptide. The two subunits have been termed "long" or subunit 1 (Sub-1) containing the major part of the active enzymatic site, and "short" or subunit 2 (Sub-2). For full activation of the enzyme, it is processed to form the pro-domain and the two sub-domains. The two subunits form a heterodimer. Based on the deduced three dimensional structure of caspase-3, it appears that the C-terminal end of the long domain as well as the N-terminus of the short sub-domain have to be freed and the C-terminus of the short subunit has to be brought into close proximity with the N-terminus of the long subunit in order to yield a correctly folded and active enzyme (Rotonda et al 1996, Mittl et al.1997, Srinivasula et al.1998).

Although pathways leading to apoptosis or necrosis have always been considered to be completely distinct, recent findings have suggested that the caspases, which represent the main mediators of apoptosis, can also be implicated in necrosis both in a negative and a positive manner. Indeed, overexpression of the caspase inhibitor CrmA in L929 cells was shown to increase by a factor of 1000 the sensitivity of these cells for the necrotic activity of TNF (Vercammen et al., 1998), indicating an inhibitory role of caspases on TNF-induced necrotic activity. Moreover, the TNFR1- and Fas-associated death domains that play a crucial role in apoptosis induction by these ligands (reviewed in Wallach et al., 1999), were recently also suggested to play an important role in necrosis induction (Boone et al., 2000). Interestingly, the FasL-induced liver necrosis was shown to be blocked by caspase inhibitors (Kunstle *et al.,* 1997).

Because caspase-mediated proteolysis is critical and central element of the apoptotic process [Nicholson D.W. and Thomberry, NA. (1997), Villa et al (1997) and Salvesen, G. S., and Dixit, V. M. (1997)], identification of the crucial downstream molecular targets of these proteases is inevitable for understanding apoptotic signal transduction. Various structural and signaling proteins have been shown to be cleaved by caspases during apoptotic death [Nicholson D.W. and Thomberry, NA. (1997), Villa, P. et al. (1997)]including ICAD, an inhibitor of caspase-activated Dnase, which is essential for internucleosomal DNA degradation but not for execution of apoptosis (Enari, M. et al. (1998) and Sakahira et al.(1998). Gelsolin, an actin-regulatory protein that modulates cytoplasmic actin gelsol transformation (Yin, H.L. and Stossel, T.P. (1979), is implicated in apoptosis on the basis of (i) its cleavage during apoptosis *in*-*vivo* [Kothakota, S. et al. (1997)](ii) prevention of apoptosis by its overexpression [Ohtsu, M. et al. (1997)] and (iii) induction of apoptosis by one of the cleaved products [Kothakota, S. et al. (1997)].

Gelsolin has Ca+2 activated multiple activities, severs actin filaments, and caps the fast growing ends of filaments, and also nucleates actin polymerization [Yin, H.L. and Stossel, T.P., (1980). Kurth, M., and Bryan, J. (1984), Janmey, P. A., and Stossel, T.P. (1987)] .

Application WO 0039160 discloses caspase-8 interacting proteins capable of interacting with Sub-1 and/or Sub-2 of caspase-8. The caspase interacting proteins were discovered by two-hybrid screen using single chain construct of caspase-8.

Typically, co-purification of Caspase-8 and caspase-8 bound proteins involve tag epitope modification of caspase-8 (e.g. HA fusion to caspase-8 Roth et al) and use of anti tag specific antibodies. However, epitope tagging of proteins may affect the activity of the tagged proteins.
caspase-8 specific antibodies are available:
Anti-Mach Cat. NO 218777, is a polyclonal chicken antibody from Calbiochem that recognizes both pro-caspase-8 and active caspase8. The immunogen used to obtain this antibody was the full length human recombinant caspase-8 protein.
Caspase-8 (D384) 6B6 is a monoclonal antibody from Cell Signaling technology, generated by immunizing mice with a synthetic peptide, KLH coupled, corresponding to residues mapping at the amino terminus of caspase-8 Sub-2. This antibody specifically detects endogenous levels of cleaved 10 kDa small sub unit of caspase 8 by western blotting. This Mab is recommended for Western blotting and does not cross react with full length caspase-8.
The B9-2 antibody is a monoclonal antibody from Pharmingen (A Becton Dickinson Company) which recognizes an 55 kDa band corresponding to caspase-8. A recombinant human caspase-8 protein fragment corresponding to amino acids 335-469 from caspase-8 was used as immunogen (Weaver et al. 2000). This monoclonal antibody is recommended to monitor the levels of caspase-8 in Western blot analysis. Identification of active caspase-8 using this Mabs may not work due that the fragment used for generating the antibodies is intact only in non-active caspase-8.
Caspase-8 p 10 (S-19):sc-6135 is an affinity-purified goat polyclonal antibody from Santa Cruz biotechnology, raised against a peptide mapping near the carboxy terminus of caspase-8 Sub-2 of human origin. This polyclonal antibody reacts with the p10 subunit (Sub-2) and precursor caspase-8 of human origin by Western blotting, immunoprecipitation and immunohistochemistry. It is non-cross-reactive with caspase-8 p20 (Sub-1).

Caspase-8 1C12 is a monoclonal antibody from Cell Signaling technology, generated by immunizing mice with a synthetic peptide, KLH coupled, corresponding to residues mapping at the carboxy terminus of caspase-8 Sub-1. This antibody specifically detects pro-caspase-8 and active caspase-8 by western blotting, but it is not capable of co-precipitating p72.

The exact sequence of the peptide used to immunize mice for the generation of this Mab is unknown.

Another antibody specific for caspase-8, designated GD-13, is provided by Sigma under the product number C2976. It is also a rabbit antibody, generated by immunizing rabbits with a synthetic peptide similar to the one according to SEQ ID No: 4. As with 1C12 from Cell Signaling, this antibody is also incapable of co-precipitating p72.

Caspase-8 p20 (H-134):sc-7890 is a rabbit polyclonal antibody from Santa Cruz biotechnology, raised against a recombinant protein corresponding to amino acids 217-350 mapping within the caspase-8 Sub-1 of human origin. This polyclonal antibody reacts with the active and precursor caspase-8 of mouse, rat and human origin by Western blotting, immunoprecipitation and immunohistochemistry.

However there is no information in technical data sheet on the efficiency by which caspase-8 is immunoprecipitated and whether a bound protein can be co-precipitated, and if so whether the recovery of caspase-8 and caspase-8 bound proteins from the immune complex is possible. Thus, currently no polyclonal and monoclonal antibodies against caspase-8 have been reported which include all the following features: are able to immunoprecipitate efficiently both pro-caspase-8 and active caspase-8 and to dissociate from the caspase-8 in the immunoprecipitate complex allowing co-purification of caspase-8 and caspase-8 bound proteins.

Therefore the method of the present invention solves a long-standing problem in the area of co-precipitation and purification of caspase-8 and caspase-8 bound proteins.

### SUMMARY OF THE INVENTION

An antibody (polyclonal, monoclonal, chimeric, fully humanized anti-anti Id antibody or fragment thereof), obtainable by immunization of an animal with a peptide from the C terminal end of the caspase-8 Sub-1 unit (e.g. CQGDNYQKGIPVETD), and fragments thereof, wherein the peptide used for immunization has the amino acid sequence CQGDNYQKGIPVETD (SEQ ID No: 4), capable of co-immunoprecipitating said caspase (both active caspase-8 and pro-caspase-8) together with the pro-caspase-8 associated protein p72 (SEQ ID No: 3), and of releasing the caspase and associated protein efficiently from the immune complex upon elution using the peptide according to SEQ ID No: 4. More specifically the peptide used for immunization may be preferably coupled to KLH.

In one embodiment, the antibody according to the invention is of the immunoglobulin isotype IgG₁.

In another embodiment, the antibody of the invention triggers processesing of caspase-8.

In one aspect, the invention provides an antibody that can be used for the development of an ELISA assay.

In another aspect, the invention provides a method for preparing an antibody according to the invention and to the use of the antibody for the method of isolation of caspase associated proteins, more preferably caspase associated protein comprising the amino acid sequence SEQ ID NO: 3, or an isoform, allelic variant, fragment, functional analogue, a fusion protein, mutant or derivative thereof, present in cell samples e.g. cell extracts, expression cDNA libraries and genomic or combinatorial peptide libraries. More specifically the invention relates to the use of said antibody for the isolation of caspase-associated proteins, wherein the caspase is caspase-8.

In one embodiment of the invention the immunogen used for immunization is linked to a carrier, preferably KLH.

The invention also provides a method for purifying a caspase, preferably caspase-8, and caspase associated proteins, which comprises contacting a material containing human caspase with said monoclonal antibody. More specifically the caspase-associated proteins are eluted, preferably with the peptide used for immunization.

In addition the invention provides the use of epitope 179 (SEQ ID:4) for obtaining an antibody according to the invention, comprising immunization of an animal with such epitope.

### BRIEF DESCRIPTION OF THE FIGURES

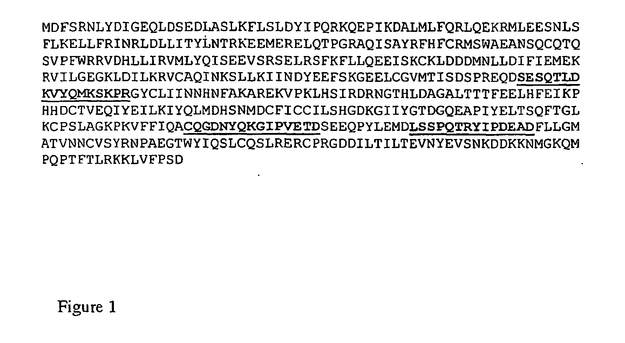

**Figure 1** shows the amino acid sequence of pro-caspase-8. The peptide sequences from caspase-8 used for the preparation of Mabs are in bold and underlined.
   Peptide 179 - The peptide CQGDNYQKGIPVETD corresponding to the C-terminus of the large subunit of caspase-8 (Sub- 1).
   Peptide 182 - The peptide LSSPQTRYIPDEAD corresponding to the N-terminus of the small subunit of the caspase-8 (Sub-2, residues Lys385-Gly399).
   Peptide 183 - The peptide SESQTLDKVYQMKSKPR corresponding to the N-terminus of Sub-1 (residues Ser217-Gly234).

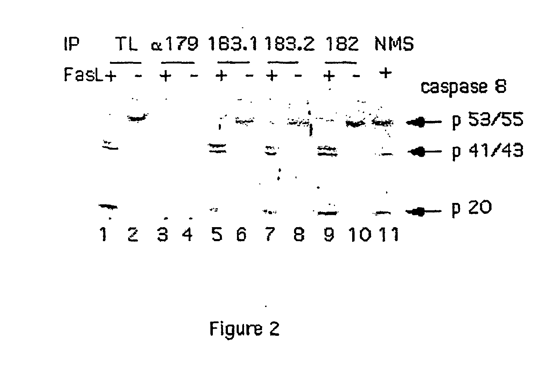

**Figure 2** shows the effective immunoprecipitation of minute amounts of caspase-8 found in lysates of BJAB cells using a monoclonal antibody against epitope 179. Depletion of caspase-8 from the BJAB cell lysates (prepared before, -, and after, +, Fas receptor stimulation) by immunoprecipitation with various antibodies is shown from left to right:
   Lanes 3 and 4, Mab 179: a monoclonal antibody prepared against a peptide corresponding to the C-terminus of Sub-1 (the large subunit of the caspase-8, residues Cys360-Asp374).
   Lanes 5 and 6, Mab 183.1 and lanes 7 and 8, Mab 183.2, two monoclonal antibodies prepared against a peptide corresponding to the N-terminus of Sub-1 (residues Ser217-Gly234).
   Lanes 9 and 10, Mab 182 a monoclonal antibody prepared against a peptide corresponding to the N-terminus of Sub-2 (the small subunit of the caspase-8) (residues Lys385-Asp399).
   Lane 11, NMS - normal mouse serum.
   The figure shows Western blotting assessment of the amounts of caspase-8 left in the cell lysates following immunoprecipitation by the indicated antibodies and in unprecipitated total cell lysates (lanes 1 and 2).

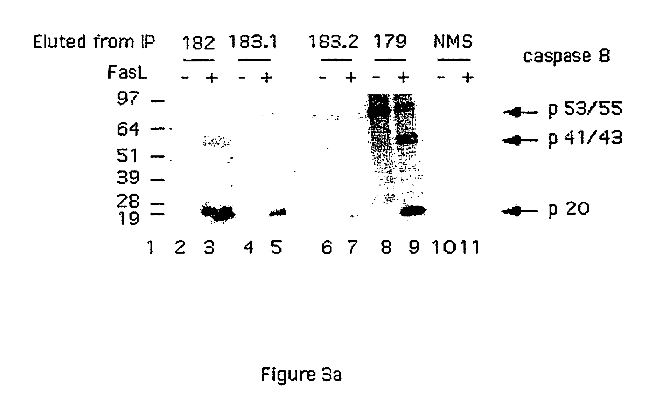

**Figure 3a** shows the elution of the caspase-8 immunoprecipitated as in Figure 2 by competing with the peptides against which the various antibodies have been raised. Caspase-8 in the eluates from the immunoprecipitates produced with the indicated antibodies is detected by Western blot analysis (as in Fig. 2).

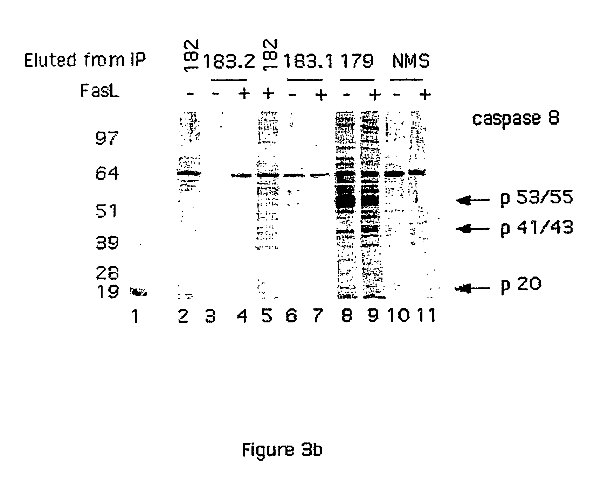

**Figure 3b** shows the elution of the caspase-8 immunoprecipitated as in Figure 2 by competing with the peptides against which the various antibodies have been raised. Caspase-8 in the eluates from the immunoprecipitates produced with the indicated antibodies are shown by Silver staining.

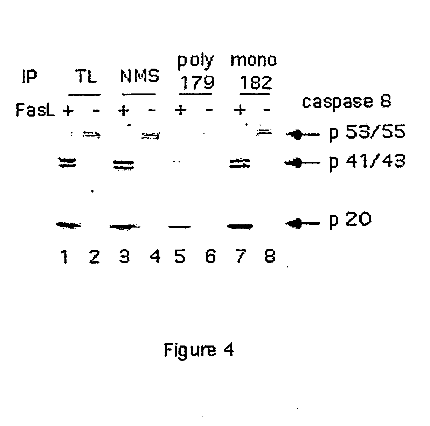

**Figure 4** shows effective immunoprecipitation of minute amounts of caspase-8 found in lysates of BJAB cells using polyclonal serum prepared by immunization with a peptide corresponding to the C-terminus of Sub-1 (the large subunit of the caspase-8, residues Cys360-Asp374). Depletion of caspase-8 from the BJAB cell lysates (prepared before, -, and after, +, Fas receptor stimulation) by immunoprecipitation with various antibodies is shown from left to right. Caspase-8 left in the lysate is detected by Western blot analysis after immunoprecipitation with the following antibodies:
   Lanes 3 and 4, NMS - normal mouse serum
   Lanes 5 and 6, anti 179 polyclonal antibody, a rabbit polyclonal antibody prepared against the C-terminus of Sub-1 (the large subunit of the caspase-8, residues Cys360-Asp374).
   Lanes 7 and 8 Mab182.
   TL-total cell lysate.

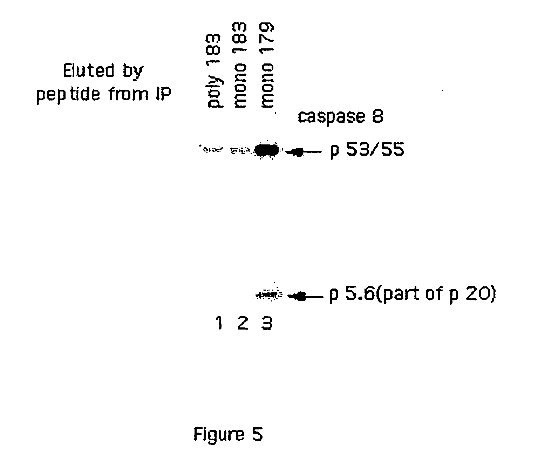

**Figure 5** shows immunoprecipitated and eluted caspase-8 from lysates of non-stimulated BJAB cells using various antibodies. Shown from left to right are the levels of caspase-8 detected by Western blot analysis after elution of immunoprecipitates carried out with the following antibodies:
   Lane 1, anti 183 polyclonal serum against the N-terminus of Sub-1 (residues Ser217-Gly234).
   Lane 2, Mab183.2, a monoclonal antibody against the N-terminus of Sub-1 (residues Ser217-Gly234).
   Lane 3, Mab179, a monoclonal antibody against the C-terminus of Sub-1 (the large subunit of the caspase-8, residues Cys360-Asp374).
   The small (5.6 kDa) fragment of caspase-8, produced by the novel-processing mode imposed by Mab179 is marked with an arrow.

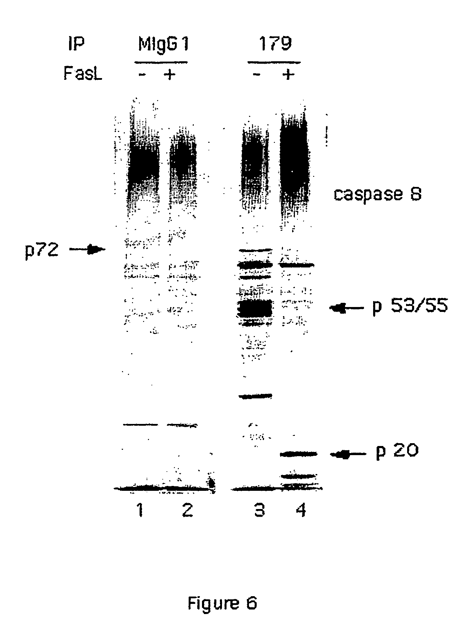

**Figure 6** shows Caspase-8 and associated protein (P72/Cari) that had been immunoprecipitated by Mab179 from lysates of Bjab cells before or after one-hour stimulation with Fas-ligand and eluted by peptide 179. Immunoprecipitated caspase-8 and associated proteins with Mab 179 were eluted (as in Fig. 3b) resolved by SDS-PAGE and Silver stained. Lanes 1 and 2 show controls in which the cell lysates were immunoprecipitated with MIgG1, mouse immunoglobulin IgG1.

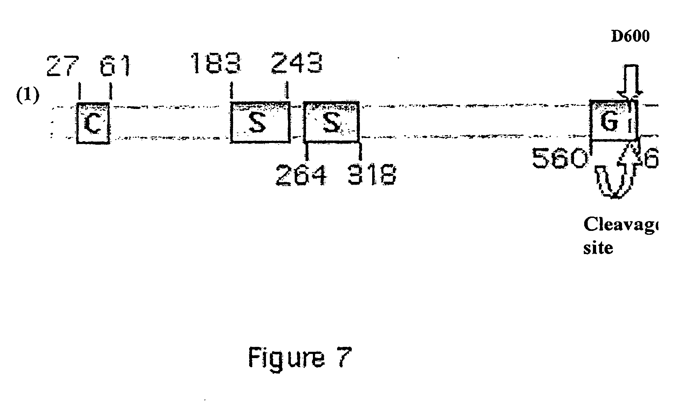

**Figure 7** shows a schematic representation of P72 protein motifs. One coiled coil motif (C) and two tandem located 'SURP motifs' (S) are located close to the N terminus of the protein, and one 'G-patch' motif is located at the C terminus of the protein (G motif). The aspartic residue D600 present inside the G motif is also indicated. D600- a mutant in which residue D600 in the protein was replaced with the glutamic acid residue.

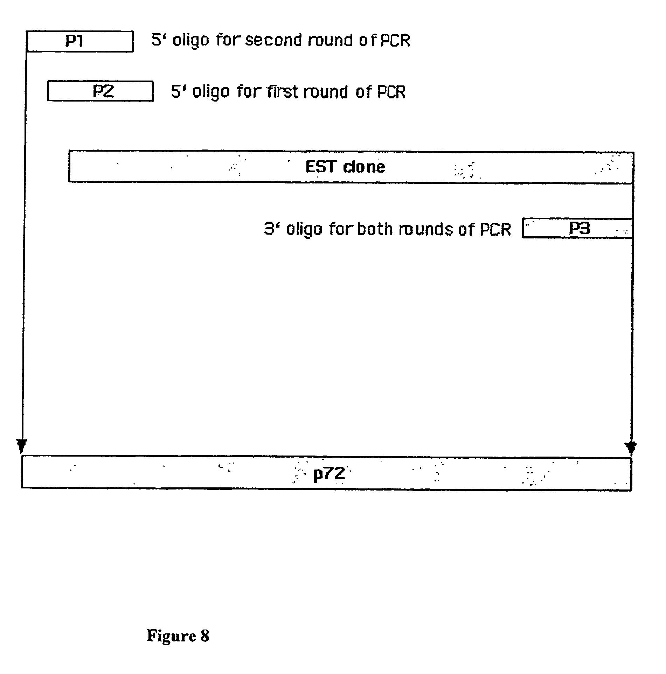

**Figure 8** shows a schematic representation of the approach used for the full-length preparation of p72 cDNA. An EST clone IMAGE 2964545 purchased from Incyte Genomics which lacks the sequence of the first 21 nucleotides (which encode the first 7 aminoacids) was used as the template for a first polymerase chain reaction (PCR) together with a pair of primers: the forward primer, P2 containing overlapping nucleic acids with the 5' EST clone and additional 15 nucleotides out of the 21 missing nucleotides and the reverse primer, P3 containing overlapping sequences with the 3' EST. The resulting PCR product was used as a template for a second PCR together with a pair of primers: the forward primer, P1 containing the whole 21 missing nucleotides and 5 nucleic acids of the EST and the reverse primer, P3 containing overlapping sequences with the 3' EST.

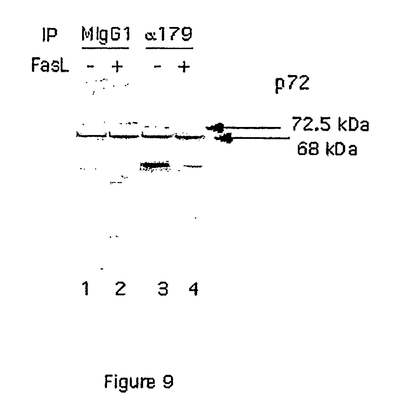

**Figure 9** shows co-immunoprecipitation of caspase-8 and p72 by Mab179 from the lysates of Bjab cells at time zero and after 20 minutes stimulation with Fas-ligand. The proteins eluted after immunoprecipitating with Mab 179 are resolved in SDS-PAGE gels and detected by Silver staining. A band with an apparent molecular weight of about 72.5 kDa corresponding to p72 is co-precipitated with pro-caspase-8 before Fas-ligand stimulation (lane 3). After 20 minutes stimulation the level of the 725 kDa band decreases and a new band corresponding to a protein of lower apparent molecular weight of about 68 kDa is detected (lane 4).
   Lane 1 and 2 show the negative controls comprising immunoprecipitation of cell lysates with MlgG1, mouse immunoglobulin IgG1.

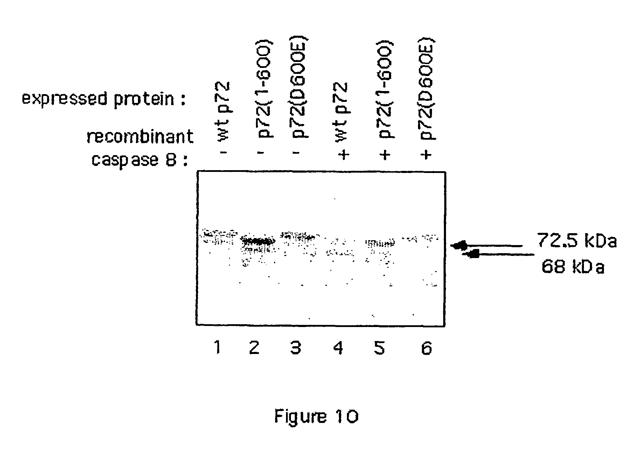

**Figure 10** shows the cleavage of p72 by active caspase-8. A protein encoded by the P72 cDNA was expressed in vitro in reticulocyte lysates in the presence of ³⁵S methionine using the TnT T7 coupled reticulocyte lysate system, and tested after incubation of 1 hour at 37°C in the presence or absence of recombinant active caspase-8. In addition the cleavage of p72 was studied with TnT products encoded by 2 different p72 cDNA mutant: one encoding p72 in which the residue D 600, suspected to be the target residue for caspase-8, was mutated to E [p72 (D600E)] and another in which the gene is deleted and the resulting truncated protein lacks the residues down-stream [D600 p72 (1-600)]. The resulting proteins were separated on SDS-PAGE and the results were visualised by phosphoimaging.

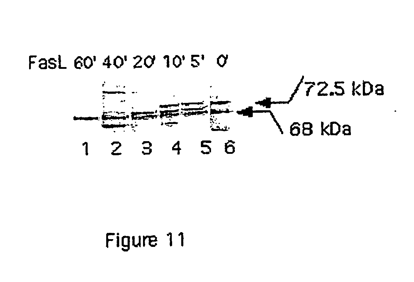

**Figure 11** shows caspase-8 and p72 that were co-imunoprecipitated by Mab179 from the lysates of Bjab cells before (0') or after 5,10, 20, 40 and 60 minutes stimulation with Fas-ligand. The peptides were eluted resolved in SDS-PAGE gels and detected by Silver staining. A peptide of apparent molecular weight of 725 kDa is detected before stimulation (0'). After 5 and 10 minutes stimulation a new protein with a lower apparent molecular weight of about 68 kDa appears. After 40 minutes stimulation the 725 kDa band completely disappears and only the 68 kDa is detected. At 60 minutes none of the above proteins mentioned were co-precipitated with caspase-8.

### DETAILED DESCRIPTION OF THE INVENTION

The present invention relates to antibodies directed against the C-terminal domain of Sub-1 of caspase-8. These, antibodies directed to this domain were found to be outstanding in their ability to immuno precipitate caspase-8 (pro-caspase-8 and active caspase) at high efficiency, even when the caspase is present at very low concentration. These antibodies, can also be used to effectively co-precipitate and isolate a protein bound to caspase-8.

The antibodies according to the invention may be polyclonal or, more preferably, monoclonal. A peptide derived from the C terminal end of Caspase-8 Sub-1, comprising the residues Cys360-Asp374 and sequence CQGDNYQKGIPVETD (peptide 179 SEQ ID: 4) was used for immunization to generate the antibody.

The peptide used for the immunization can be synthesized purified by reverse HPLC and coupled to any carrier such as KLH, preferably through its natural cystein or through an artificially fused cystein, to expose the peptide to the surface of the carrier, and used to immunize e.g. mice for the preparation of monoclonal antibodies and rabbit for polyclonal antibodies.

In one embodiment the co-immunoprecipitation of caspase-8 and caspase-8 bound proteins is carried out using a caspase-8 antibody specific to the epitope 179 found at the C-terminal domain of Sub-1.

The co-immunoprecipitation according to the invention, is carried on samples selected from cell lysates from resting or stimulated cells, or from cDNA expression libraries, from genomic or combinatorial peptide libraries.

The cells can be stimulated, prior to lysis and immunoprecipitation, using different apoptosis inducing agents, such as treatment with lymphokines, for example Fas-ligand, TNF, or by environmental factors such as starvation, heat shock etc.

Using the antibodies in co-immunoprecipitation according to the invention, caspase-8 and the caspase-bound protein could be efficiently eluted from the immunoprecipitate complex and recovered in the supernatant by competition with the peptide derived from caspase-8 CQGDNYQKGIPVETD, the same peptide used for immunization.

Peptide 179 or epitope 179 from caspase-8 of sequence CQGDNYQKGIPVETD and SEQ ID NO:4, or a mutein, fragment thereof, fusion protein, or derivative thereof is used for immunizing and generating antibodies according to the invention. The peptide for immunization can be produced, by chemical synthesis as described above, or by recombinant DNA technology in mammalian cells, or by cleavage of a purified protein. The protein may also be produced in bacterial or insect cells as detailed in Current Protocols in Molecular Biology, by F. M. Ausubel, ISBN: 047150338X,1988 chapter 16.

Not comprised in the invention are muteins of the above epitope 179 (SEQ ID NO:4) of the invention, which muteins retain essentially the same properties of the peptide having essentially only the naturally occurring sequences of the peptide. Such "muteins" may be ones in which amino acid residues may be deleted, added or substituted by others in the peptide, such that modifications of this kind do not substantially change the biological properties of the peptide mutein with respect to the peptide itself.

These muteins are prepared by known synthesis and/or by site-directed mutagenesis techniques, or any other known technique suitable therefor.

Preferred changes for muteins are what are known as "conservative" substitutions. Conservative amino acid substitutions include synonymous amino acids within a group which have sufficiently similar physicochemical properties that substitution between members of the group will preserve the biological function of the molecule, Grantham, Science, Vol.185, pp. 862-864 (1974). It is clear that insertions and deletions of amino acids may also be made in the above -defined sequences without altering their function, particularly if the insertions or deletions only involve a few amino acids, e.g., under 3, and preferably under 2, and do not remove or displace amino acids which are critical to a functional conformation

Preferably, the synonymous amino acid groups are those defined in Table I. More preferably, the synonymous amino acid groups are those defined in Table II; and most preferably the synonymous amino acid groups are those defined in Table III.

**TABLE I Preferred Groups of Synonymous Amino Acids**

| Amino Acid | Synonymous Group |
|---|---|
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser. Ala, Gly, His, Gln, Thr |
| Ala | Gly, Thr, Pro, Ala |
| Val | Met, Tyr, Phe, Ile, Leu, Val |
| Gly | Ala, Thr; Pro, Ser. Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gln | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

**TABLE II More Preferred Groups of Synonimous Amino Acids**

| Amino Acid | Synonymous Group |
|---|---|
| Ser | Ser |
| Arg | His, Lys, Arg |
| Leu | Ile, Phe, Met, Leu |
| Pro | Ala, Pro |
| Thr | Thr |
| Ala | Pro, Ala |
| Val | Met, Ile, Val |
| Gly | Gly |
| Ile | Ile, Met, Phe, Val, Leu |
| Phe | Met, Tyr, Ile, Leu, Phe |
| Tyr | Phe, Tyr |
| Cys | Ser, Cys |
| His | Arg, Gln, His |
| Gln | Glu, His, Gln |
| Asn | Asp, Asn |
| Lys | Arg, Lys |
| Asp | Asn, Asp |
| Glu | Gln, Glu |
| Met | Phe, Ile, Val, Leu, Met |
| Trp | Trp |

**TABLE III Most Preferred Groups of Synonymous Amino Acids**

| Amino Acid | Synonymous Group |
|---|---|
| Ser | Ser |
| Arg | Arg |
| Leu | Ile, Met, Leu |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Ser, Cys |
| His | His |
| Gln | Gln |
| Asn | Asn |
| Lys | Lys |
| Asp | Asp |
| Glu | Glu |
| Met | Ile, Leu, Met |
| Trp | Trp |

Examples of production of amino acid substitutions in proteins which can be used for obtaining muteins of the protein include any known method steps, such as presented in US patents RE 33,653, 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Straw et al).

The protein or peptide is then purified from the synthetic mixture or from the cells in which it has been produced. Protein purification methods are known to the person of skill in the art and are detailed e.g., in the above-noted Current Protocols in Molecular Biology, chapter 16, and in Current Protocols in Protein Science, Wiley and Sons Inc. chapters 5 and 6. Advantageously, the peptide may be produced as a fusion with KLH or Glutathione-S-transferase or the like, or a sequence tag, such as the histidine tag sequence. The use of fusion or tagged proteins simplifies the purification procedure, as detailed in the above-noted Current Protocols in Molecular Biology, chapter 16, and in the instructions for the above-noted Qiagen his-tag protein expression and purification kit.

If the protein or peptide has been expressed as a fusion protein, it the fusion partner could be cleaved before using the protein for the generation of antibodies, in order to avoid generation of antibodies against the fusion partner. The cleavage of fusion partners and the isolation of the desired protein are described in the above-noted Current Protocols in Molecular Biology, chapter 16. Vectors, protocols and reagents for expressing and purifying maltose-binding protein fused recombinant proteins are also available commercially.

When producing a peptide, it may be desirable not to remove the fusion partner, as the fusion protein may stimulate the production of antibodies against the peptide.

As noted further above, peptide may also be synthesized by chemical methods known in the art of chemistry.

The generation of polyclonal antibodies against proteins is described chapter 2 of Current Protocols in Immunology, Wiley and Sons Inc. The generation of antibodies against peptides may necessitate some changes in protocol, because of the generally lower antigenicity of peptides when compared to proteins. The generation of polyclonal antibodies against peptides is described in the above-noted Current Protocols in Immunology, chapter 9.

Monoclonal antibodies may be prepared from B cells taken from the spleen or lymph nodes of immunized animals, in particular rats or mice, by fusion with immortalized B cells under conditions, which favours the growth of hybrid cells. For fusion of murine B cells, the cell line Ag-8 is preferred.

The technique of generating monoclonal antibodies is described in many articles and textbooks, such as Current Protocols in Immunology, Wiley and Sons Inc. chapter 2. Chapter 9 therein describes the immunization, with peptides, or animals. Spleen or lymph node cells of these animals may be used in the same way as spleen or lymph node cells of protein-immunized animals, for the generation of monoclonal antibodies as described in chapter 2 therein.

The techniques used in generating monoclonal antibodies are further described in Kohler and Milstein, (1975), and in USP 4,376,110.

The preparation of antibodies from a gene bank of human antibodies the hyper variable regions thereof are replaced by almost random sequences is described in USP 5,840,479. Such antibodies are preferred if it is difficult to immunize an animal with a given peptide or protein. Some structures are poorly immunogenic and may remain so despite of the addition of adjuvants and of linking to other proteins in fusion constructs. The antibodies described in USP 5,840,479 are further preferred if it is desired to use antibodies with a structure similar to human antibodies, for instance, when antibodies are desired that have a low immunogenicity in humans.

Once a suitable antibody has been identified, it may be desired to change the properties thereof. For instance, a chimeric antibody may achieve higher yields in production. Chimeric antibodies wherein the constant regions are replaced with constant regions of human antibodies are further desired when it is desired that the antibody be of low immunogenicity in humans. The generation of chimeric antibodies is described in a number of publications, such as Cabilly et al., 1984, Morrison et al., 1984, Boulianne et al, 1984, EP 125023, EP 171496, EP 173494, EP 184187, WO 86/01533, WO 87/02671, and Harlow and Lane, Antibodies: A Laboratory Manual, Cold Spring harbor Laboratory,1988.

"Fully humanized antibodies" are molecules containing both the variable and constant region of the human immunoglobulin. Fully humanized antibodies can be potentially used for therapeutic use, where repeated treatments are required for chronic and relapsing diseases such as autoimmune diseases. One method for the preparation of fully human antibodies consist of "humanization" of the mouse humoral immune system, i.e. production of mouse strains able to produce human Ig (Xenomice), by the introduction of human immunoglobulin (Ig) loci into mice in which the endogenous Ig genes have been inactivated. The Ig loci are exceedingly complex in terms of both their physical structure and the gene rearrangement and expression processes required to ultimately produce a broad immune response. Antibody diversity is primarily generated by combinatorial rearrangement between different V, D, and J genes present in the Ig loci. These loci also contain the interspersed regulatory elements, which control antibody expression, allelic exclusion, class switching and affinity maturation. Introduction of unrearranged human Ig transgenes into mice has demonstrated that the mouse recombination machinery is compatible with human genes. Furthermore, hybridomas secreting antigen specific humAbs of various isotypes can be obtained by Xenomice immunization with antigen.

Fully humanized antibodies and methods for their production are known in the art (Mendez et al., Nature Genetics 15:146-156 (1997);Buggemann et al., EurJ. Immunol. 21:1323-1326 (1991); Tomizuka et al., *Proc. Natl. Acad. Sci. USA* 97:722-727 (2000) Patent WO 98/24893.

Another type of antibody is an anti-idiotypic antibody. An anti-idiotypic (anti-Id) antibody is an antibody, which recognizes unique determinants generally associated with the antigen-binding site of an antibody. An Id antibody can be prepared by immunizing an animal of the same species and genetic type (e.g. mouse strain) as the source of the Mab to which an anti-Id is being prepared. The immunized animal will recognize and respond to the idiotypic determinants of the immunizing antibody by producing an antibody to these idiotypic determinants (the anti-Id antibody). See, for example, U.S. Patent No. 4,699,880.

The anti-Id antibody may also be used as an "immunogen" to induce an immune response in yet another animal, producing a so-called anti-anti-Id antibody. The anti-anti-Id may be epitopically identical to the original mAb, which induced the anti-Id. Thus, by using antibodies to the idiotypic determinants of a mAb, it is possible to identify other clones expressing antibodies of identical specificity.

Accordingly, Mabs generated against the C-terminal Sub-unit of caspase-8 , analogs, fragments or derivatives thereof, of the present invention may be used to induce anti-Id antibodies in suitable animals, such as BALB/c mice. Spleen cells from such immunized mice are used to produce anti-Id hybridomas secreting anti-Id mAbs. Further, the anti-Id mAbs can be coupled to a carrier such as keyhole limpet hemocyanin (KLH) and used to immunize additional BALB/c mice. Sera from these mice will contain anti-anti-Id antibodies that have the binding properties of the original mAb specific for an epitope of the above caspase-8, or analogs, fragments and derivatives thereof.

The anti-Id mAbs thus have their own idiotypic epitopes, or "idiotopes" structurally similar to the epitope being evaluated.

The term "antibody" is also meant to include both intact molecules as well as fragments thereof, such as, for example, Fab and F (ab') 2, which are capable of binding antigen. Fab and F (ab') 2 fragments lack the Fc fragment of intact antibody, clear more rapidly from the circulation, and may have less non-specific tissue binding than an intact antibody (Wahl et al.,1983).

It will be appreciated that Fab and F (ab') 2 and other fragments of the antibodies useful in the present invention may be used for the detection and quantitation of the p72 protein according to the methods disclosed herein for intact antibody molecules. Such fragments are typically produced by proteolytic cleavage, using enzymes such as papain (to produce Fab fragments) or pepsin (to produce F (ab') 2 fragments).

An antibody is said to be "capable of binding" a molecule if it is capable of specifically reacting with the molecule to thereby bind the molecule to the antibody. The term "epitope" is meant to refer to that portion of any molecule capable of being bound by an antibody, which can also be recognized by that antibody. Epitopes or "antigenic determinants" usually consist of chemically active surface groupings of molecules such as amino acids or sugar side chains and have specific three-dimensional structural characteristics as well as specific charge characteristics.

An "antigen" is a molecule or a portion of a molecule capable of being bound by an antibody, which is additionally capable of inducing an animal to produce antibody capable of binding to an epitope of that antigen. An antigen may have one or more than one epitope. The specific reaction referred to above is meant to indicate that the antigen will react, in a highly selective manner, with its corresponding antibody and not with the multitude of other antibodies, which may be evoked by other antigens.
The epitope 179 of the invention can be used to develop specific polyclonal, monoclonal antibodies for the outstanding immunoprecipitation and elution of caspase-8 and caspase-8 associated proteins. The antibodies can be also used to induce a novel-processing of caspase-8.

The antibodies developed to the C-terminal domain of Sub-1 such as Mab 179 can be used in combination with antibodies developed to the N-terminal domain of Sub-1 such as Mab 183 to specifically isolate caspase-8 regulatory proteins. The C-terminal domain of Sub-1 is known to be part of the active site of caspase-8 and therefore regulatory proteins may bind to it (Thornberry et al. 1997). Co-precipitating caspase-8 and regulatory proteins associated with Mab 183 first and then applying Mab 179 may release the regulatory proteins from caspase-8 to the medium and lead to the identification of key proteins involved in apoptosis regulation.

In one embodiment, Cari, (p 72, SEQ ID NO:3) a pro-caspase-8 binding protein, was isolated using the immunoprecipitation of the invention with Mab 179. Cari was found to be cleaved by active caspase-8 and to be involved in caspase-8 activation and apoptosis.

The antibodies (or fragments thereof) useful in the present invention may be employed histologically, as in immunofluorescence or immunoelectron microscopy, for *in situ* detection of caspase-8. *In situ* detection may be accomplished by removing a histological specimen from a patient, and providing the labeled antibody of the present invention to such a specimen. The antibody (or fragment) is preferably provided by applying or by overlaying the labeled antibody (or fragment) to a biological sample. Using the present invention, those of ordinary skill will readily perceive that any of wide variety of histological methods (such as staining procedures) can be modified in order to achieve such *in situ* detection.

The biological sample may be treated with a solid phase support or carrier such as nitrocellulose, or other solid support or carrier, which is capable of immobilizing cells, cell particles or soluble proteins. The support or carrier may then be washed with suitable buffers followed by treatment with a detectably labeled antibody in accordance with the present invention, as noted above. The solid phase support or carrier may then be washed with the buffer a second time to remove unbound antibody. The amount of bound label on said solid support or carrier may then be detected by conventional means.

By "solid phase support", "solid phase carrier", "solid support", "solid carrier", "support" or "carrier" is intended any support or carrier capable of binding antigen or antibodies. Well-known supports or carriers include glass, polystyrene, polypropylene, polyethylene, dextran, nylon amylases, natural and modified celluloses, polyacrylamides, gabbros and magnetite. The nature of the carrier can be either soluble to some extent or insoluble for the purposes of the present invention. The support material may have virtually any possible structural configuration so long as the coupled molecule is capable of binding to an antigen or antibody. Thus, the support or carrier configuration may be spherical, as in a bead, cylindrical, as in the inside surface of a test tube, or the external surface of a rod. Alternatively, the surface may be flat such as a sheet, test strip, etc. Preferred supports or carriers include polystyrene beads. Those skilled in the art will know may other suitable carriers for binding antibody or antigen, or will be able to ascertain the same by use of routine experimentation.

The binding activity of a given lot of antibody, of the invention as noted above, may be determined according to well-known methods. Those skilled in the art will be able to determine operative and optimal assay conditions for each determination by employing routine experimentation.

Other such steps as washing, stirring, shaking, filtering and the like may be added to the assays as is customary or necessary for the particular situation.

One of the ways in which an antibody in accordance with the present invention can be detectably labeled is by linking the same to an enzyme and used in an enzyme immunoassay (EIA). This enzyme, in turn, when later exposed to an appropriate substrate, will react with the substrate in such a manner as to produce a chemical moiety, which can be detected, for example, by spectrophotometric, fluorometric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholin-esterase. The detection can be accomplished by colorimetric methods, which employ a chromogenic substrate for the enzyme. Detection may also be accomplished by visual comparison of the extent of enzymatic reaction of a substrate in comparison with similarly prepared standards.

Detection may be accomplished using any of a variety of other immunoassays. For example, by radioactive labeling the antibodies or antibody fragments, it is possible to detect R-PTPase through the use of a radioimmunoassay (RIA). A good description of RIA may be found in Laboratory Techniques and Biochemistry in Molecular Biology, by Work, T.S. et al., North Holland Publishing Company, NY (1978) with particular reference to the chapter entitled "An Introduction to Radioimmune Assay and Related Techniques" by Chard, T.. The radioactive isotope can be detected by such means as the use of a g counter or a scintillation counter or by autoradiography.

It is also possible to label an antibody in accordance with the present invention with a fluorescent compound. When the fluorescent labeled antibody is exposed to light of the proper wavelength, its presence can be then detected due to fluorescence. Among the most commonly used fluorescent labeling compounds are fluorescein isothiocyanate, rhodamine, phycoerythrine, pycocyanin, allophycocyanin, o-phthaldehyde and fluorescamine.

The antibody can also be detectably labeled using fluorescence emitting metals such as ¹⁵²E, or others of the lanthanide series. These metals can be attached to the antibody using such metal chelating groups as diethylenetriamine pentaacetic acid (ETPA).

The antibody can also be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

An antibody molecule of the present invention may be adapted for utilization in an immunometric assay, also known as a "two-site" or "sandwich" assay. In a typical immunometric assay, a quantity of unlabeled antibody (or fragment of antibody) is bound to a solid support or carrier and a quantity of detectably labeled soluble antibody is added to permit detection and/or quantitation of the ternary complex formed between solid-phase antibody, antigen, and labeled antibody.

Typical, and preferred, immunometric assays include "forward" assays in which the antibody bound to the solid phase is first contacted with the sample being tested to extract the antigen from the sample by formation of a binary solid phase antibody-antigen complex. After a suitable incubation period, the solid support or carrier is washed to remove the residue of the fluid sample, including un-reacted antigen, if any, and then contacted with the solution containing an unknown quantity of labeled antibody (which functions as a "reporter molecule"). After a second incubation period to permit the labeled antibody to complex with the antigen bound to the solid support or carrier through the unlabeled antibody, the solid support or carrier is washed a second time to remove the un-reacted labeled antibody.

In another type of "sandwich" assay, which may also be useful with the antigens of the present invention, the so-called "simultaneous" and "reverse" assays are used. A simultaneous assay involves a single incubation step as the antibody bound to the solid support or carrier and labeled antibody are both added to the sample being tested at the same time. After the incubation is completed, the solid support or carrier is washed to remove the residue of fluid sample and un-complexed labeled antibody. The presence of labeled antibody associated with the solid support or carrier is then determined as it would be in a conventional "forward" sandwich assay.

In the "reverse" assay, stepwise addition first of a solution of labeled antibody to the fluid sample followed by the addition of unlabeled antibody bound to a solid support or carrier after a suitable incubation period is utilized. After a second incubation, the solid phase is washed in conventional fashion to free it of the residue of the sample being tested and the solution of un-reacted labeled antibody. The determination of labeled antibody associated with a solid support or carrier is then determined as in the "simultaneous" and "forward" assays.

The creation of immunoassays, such as RIA or ELISA, has been described in many articles, textbooks, and other publications. Reference is made to WO 97/03998, p. 48, line 4 to p. 52, line 27. Immunoassays of the invention may be if two general types: Firstly, immunoassays using a immobilized caspase, or an equivalent peptide, may be used in the quantification of caspase-8. Secondly, immunoassays using immobilized antibodies directed against an epitope of a caspase may be used to quantify caspase proteins.

Such assays may find use in diagnostics, as the level of caspase and of other proteins involved in apoptotic pathways may need to be evaluated in a number of disorders or syndromes where involvement of such pathways is a possibility.

In one embodiment, a pro-caspase-8 interacting protein, denoted Cari (p 72, SEQ ID NO: 3) was isolated with the help of antibodies according to the invention. However, if one desires to immunoprecipitate proteins bound to a different caspase, using the above immunoprecipitation method, an antibody specific to the C-terminal domain of the Sub-1 of a caspase other than caspase-8 can be used.

Since antibodies according to the invention are able to precipitate either pro-caspase-8 and caspase-8, caspase binding proteins could be co-precipitated together with active caspase or pro-caspase by co-immunoprecipitation.

The invention will be now illustrated by the following non-limiting examples.

### EXAMPLES

### Example 1:

### Immunization of mice for generation of monoclonal antibodies specific to caspase-8.

Following activation, caspase-8 is cleaved and assembled in two sub units (Sub-1 and Sub-2).

For the generation of antibodies specific to new possible epitopes formed following caspase-8 activation, synthetic peptides derived from the C-terminus of Sub-1 and N-terminus of Sub-1 and Sub-2 were used to immunize mice.

The following peptides were used to immunize mice for the generation of monoclonal antibodies:
Peptide 179 -The peptide CQGDNYQKGIPVETD (SEQ ID:4) corresponding to the C-terminus of the large subunit of caspase-8 (Sub- 1), (epitope corresponding to residues Cys360-Asp374 Fig. 1) was synthesized purified by reverse HPLC and coupled to the carrier KLH trough its natural cystein, to expose the peptide to the surface of the carrier.
Peptide 182 - The peptide LSSPQTRYIPDEADC (SEQ ID:5)corresponding to the N-terminus of the small subunit of the caspase-8 (Sub-2, residues Lys385-Gly399) was synthesized purified by reverse HPLC and coupled to carrier KLH trough the C which is not derived from the sequence of Sub-2.
Peptide 183 - The peptide SESQTLDKVYQMKSKPRC (SEQ ID:6) corresponding to the N-terminus of Sub-1 (residues Ser217-G1y234), was synthesized purified by reverse HPLC and coupled to carrier KLH trough the C which is not derived from the sequence of Sub-1.

Four immunizations and two boosts with the same amount of antigen (peptide-KLH) were administered to mice as follows:

First immunization with 50 µg of peptide-KLH were dissolved in 50µl PBS and homogenised with 50µl complete Freund's Adjuvant and injected into the footpad of each of five 7 week old Balb/C female mice.

For the second immunization, carried out 2 weeks after the first immunization, mice were intramuscularly boosted with the same amount of the peptide in a 50 % (v/v) solution of incomplete Freund's adjuvant.

For the third immunization, carried out two weeks after the second immunization, mice were injected intraperitoneal with 50 µg of peptide-KLH in 50µl PBS.

Sera of the injected mice were tested 10 days after the second and the third immunization.

The fourth immunization (carried only for peptides 182 and 183), was performed a month latter in similar way as the third immunization.

One month after the fourth immunization (or third immunization for mice challenged with peptide 179) two boosts were carried out (in a similar way as the third and fourth immunization) within two-day interval.

Four days latter the spleen and inguinal lymph nodes of the two mice exhibiting the highest specific immunoreactivity were taken for fusion with myeloma cells (Eshhar Z, 1985).

### Example 2:

### Immunization of rabbits for generation of polyclonal antibodies specific to caspase-8.

Rabbits were immunized with 179-KLH and 183-KLH for the generation of specific polyclonal antibodies.

The first immunization was carried out with 100µg of peptide-KLH which was dissolved in 50µl PBS and homogenised with 50µl complete Freund's Adjuvant and injected sub-cutaneously. A second immunization was carried out two weeks later with the same amount of peptide-KLH and injected intramuscularly two weeks later with incomplete Freund's adjuvant. These two immunizations were followed by two boosts of the same amount of peptide-KLH dissolved in PBS and administered sub-cutaneously at two weeks interval.

### Example 3:

### Hybridoma preparation, selection of antibody producing clones and purification of antibodies from ascitis fluids.

The fusion process and hybridoma cell selection were performed according to the protocols in Eshhar Z, 1985. Briefly, a mixture of spleen and lymph node cells from 2 reactive mice 110x10⁶ were fused with 32x10⁶ NSO/1 myeloma variant myeloma cells by a short incubation with PEG. The PEG was first slowly diluted with DMEM and then completely removed by centrifugation. The cells were re-suspended in DMEM-HAT medium, distributed in 96 wells plates at a concentration of about 25x10⁴ cells/well and incubated in an 8% CO₂ incubator at 37 °C. The medium in all the hybridoma wells was changed to DMEM supplemented with 10% Horse Serum (HS). Hybridoma culture supernatant samples were screened for the presence of specific Mabs two weeks after the fusion by ELISA (described in Example 12 below). Cells from wells, in which the presence of specific antibodies was detected in the culture supernatant, were transferred to 24 well plates. Positive cells were subcloned twice; at this stage all the sub-clones were found to be positive. The clones were expanded in 24 wells and then to 25 cm² T-flasks. The expanded cultures were monitored for secretion of specific Mabs. Ampoules of cells from positive cultures were frozen and stored in liquid nitrogen.

Out of approximately 700 clones screened for detecting specific antibodies to peptide 179 only one positive clone was found (Mab 179), out of 700 clones screened for detecting specific antibodies to peptide 182 only 1 positive clone was found (Mab 182) and out of 1100 clones screened for detecting specific antibodies to peptide 183 only 2 positive clones were found (Mabs 183.1 and 183.2). The positive clones were sub-cloned by limiting dilution in 96 well plates. Supernatants from the growing clones were tested several times for specific antibodies by ELISA (described in example 12).

Positive hybridoma clones were grown in tissue culture flasks in DMEM containing 15% horse serum and ampoules were frozen from part of the cultures. In parallel, cells of different hybridoma clones were injected, to 2-4 mice each, to obtain ascites fluids. The antibodies were purified from ascites fluid by affinity purification using affigel beads (affigel 15 Biorad) cross-linked with BSA (Pierce Cat 77116) coupled to the synthetic peptide used for mice immunization (peptides 179,182 or 183).

For antibody purification, ascites precipitated by 50% ammonium sulphate was dialyzed against PBS for 16 hours at 0°C. Following dialysis, aliquots were incubated with 1 ml affigel-BSA-peptide beads for 16 hours at 0°C and the pre-incubated beads were used to pack a 1 ml column. Initially the column was washed with 10 ml PBS, followed by a wash with 10mM Tris pH 75 containing 1 M NaCl and a wash with PBS. The antibodies were eluted from the column with a solution containing 100mM glycine HCl pH 2.7 and 0.5M NaCl. 1 ml fractions were collected in tubes containing 40µl Tris base for the neutralization of the eluant. From 25 ml ascites about 5-13.6 mg-purified antibodies was obtained.

### Example 4:

### Monoclonal antibodies isotype.

The isotype of monoclonal antibodies was determined using a commercial isotyping kit (Southern Biotechnology Associates, INC cat 5300-05) according to the manufacturer's assay procedure. Mabs 183 and 179 were identified as IgG1, whereas Mab 182 was found to be of the IgM class.

### Example 5:

### Immunoprecipitation of caspase-8 with Mabs 179, 182 and 183.

The different monoclonal anti-caspase-8 antibodies described in the example 3 above were tested for their capacity to immunoprecipitate caspase-8 (see example 12 below) from lysates of resting and activated Bjab cells. Bjab line is a continuous lymphoma cell line derived from the African case of Burkitt's lymphoma (Clements GB et al.1975).

Bjab cells were stimulated with Fas-ligand for one hour. Cell lysates were prepared from Bjab cells before and after stimulation. Following immunoprecipitation with Mabs 179, 182 and 183 (as described in example 12) the "depleted lysate" and the caspase-8 eluted with the corresponding peptides were analysed by SDS-PAGE and Silver staining or by Western blot analysis using anti Sub-1 antibody as the first antibody (Cell Signaling Technology Caspase-8 ICI2 Cat 9746).

Figure 2 shows a Western blot analysis (performed as described in example 10 below) of total cell extracts and "depleted lysates", obtained after immunoprecipitation with Mabs 179,183.1 and 183.2 and 182.

In non-stimulated cells (lanes 2, 4, 6, 8 and 10), a band doublet corresponding to pro-caspase-8 isoform α1 and α2 (pro-caspase-8 53/55 kDa) was detected in total cell extracts (lane 2) and in depleted lysates obtained with anti 183 and anti 182 antibodies (lanes 6, 8 and 10) in contrast no pro-caspase-8 was detected in depleted lysates obtained with Mab 179 (lane 4).

In stimulated cells the levels of pro-caspase-8 in total cell extract were lower (lane 1). Additional smaller bands corresponding to activated caspase-8 fragments appeared upon activation i.e. a doublet of partially processed caspase-8 corresponding to isoform α1 and α2 (partially processed caspase-8 p 41/43, lacking Sub-2) and a smaller band corresponding to Sub-1 (p 20). Depletion of the minute amounts of pro-caspase-8 and activated caspase-8 fragments by the Mabs was tested on lysates of Fas-ligand stimulated cells (Fig. 2 lanes 3, 5, 7, 9 and 11).

It should be noted that depletion of Sub-1 by Mab 182, specific to Sub-2, was also tested since activated caspase-8 comprises Sub-1 bound to Sub-2 and therefore removal of Sub-2 by immunoprecipitation with Mab 182 should consequently lead to depletion of Sub-1.

Immunoprecipitation of caspase-8 from stimulated cell lysates show that Mabs 182, 183.1 and 183.2, similar to the normal mouse serum control (Fig. 2 lane 11), did not remove the small amounts of remaining pro-caspase-8 or the active caspase8 fragments (lanes 9, 7, 5 and 11 respectively). In contrast to these results, treatment of the cell lysates with Mab 179 (lane 3), efficiently removed all the pro-caspase-8 as well as the active caspase-8 fragments.

Figures 3a (Western blot analysis) and 3b (protein detection by Silver staining) show that immunoprecipitated pro-caspase-8 and active caspase-8 fragments by Mabs 179, 182 and 183.1 and 183.2 antibodies could be efficiently recovered into the supernatant by competition with the respective peptides against which the various antibodies were been raised (example 12).

In non-stimulated cells (Figs. 3a lanes 2, 4, 6, 8 and 10 and Fig. 3b lanes 2, 3, 6, 8, and 10) pro-caspase-8 is efficiently recovered by immunoprecipitation with Mab 179 and competition with peptide 179 (Figs 3a and 3b lane 8). In stimulated cells, in spite of the small amount of pro-caspase-8 left after activation immunoprecipitation with Mab 179 resulted in effective recovery of the protein (Figs. 3a and 3b lane 9). Some recovery of pro-caspase-8 could be observed in non activated cells by Mab 183.2 (Fig. 3a lane 6) and in activated cells by Mab 183.1 (Fig. 3a lane 5) where active fragments of caspase-8 could be recovered in lysates of activated cells by Mabs 182 (Fig. 3a lane 3), 183.1 (Fig. 3a lane 5) and 183.2 (Fig.3a lane 7 only p20).

The results obtained indicated that the Mab 179 developed against the peptide corresponding to the C-temninus of Sub-1 (179 epitope) is very efficient for immunoprecipitation and purification of pro-caspase-8, even present in trace amounts, as well as for activated caspase-8.

Polyclonal antibody specific to the same 179 epitope (prepared as described in example 1 above) was generated to investigate whether the 179 epitope has the unique capability of eliciting antibodies, which can be generally used for the efficient immunoprecipitation and purification of pro-caspase-8 and active caspase-8. The "depleted lysates" obtained by immunoprecipitation with polyclonal antibody specific to epitope 179 (lanes 5 and 6 for activated and non-activated cells respectively) or by monoclonal antibody specific to epitope 182 (lanes 7 and 8 for activated and non-activated cells respectively) were compared. The results in Figure 4 clearly show that indeed, pro-caspase-8 and caspase-8 fragments from stimulated cell lysates are more efficiently removed from the cell lysate by polyclonal anti 179 antibody than by monoclonal anti182 antibody.

In parallel immunoprecipitation and recovery of pro-caspase-8 from resting cells lysates carried out with Mab 183 and polyclonal antibody specific to the 183 epitope (described in example 1) were compared to those obtained with Mab 179. Figure 5 shows that immunoprecipitation of pro-caspase-8 by Mab 183 and poly 183 is ineffective while immunoprecipitation of pro-caspase-8 by Mab 179 is remarkably superior.
An additional caspase-8 derived fragment of about 5.6 kDa is observed only in immunoprecipitates carried with Mab 179 (lane 3). Antibodies developed against the region of caspase-8 that corresponds to the C-terminus of the large caspase Sub-1 have a unique ability to impose on the caspase a novel mode of processing.

The results observed above indicate that epitope 179 of caspase-8, unlike other epitopes, has the special capability of eliciting specific antibodies which are very efficient for immunoprecipitation of pro-caspase-8 and activated caspase-8 and are able to induce pro-caspase-8 autoprocessing.

### Example 6:

### Isolation and identification of a caspase-8 binding protein (p72).

Due to its capability to efficiently immunoprecipitate caspase-8, Mab 179 was exploited to co-immunoprecipitate caspase-8 and caspase-8-associated proteins.

Bjab cells (Steinitz M, Klein G. 1975) were stimulated with Fas-ligand for one hour and cell lysates were prepared from cells before and after stimulation. Following immunoprecipitation and elution, as described in example 12, the recovered proteins were resolved by SDS-PAGE and detected by Silver-staining. Immunoprecipitation with mouse IgG1 served as the negative control. The results in Fig. 6 show that a protein of an apparent molecular weight of about 72.5 kDa (herein called p72) is co-precipitated with pro-caspase-8 (p 53/55) in lysates from resting cells (lane 3), but not with active caspase-8 in lysates from stimulated cells (lane 4).

In addition, a p72 protein was found to co-immunoprecipitate with pro-casapse-8 also in lysates prepared from non-stimulated HeLa, Raji, H9, K562, HL-60, CEM and Hut78 cells (ATCC).

These results suggest that a protein, p72, is generally bound to pro-caspase-8 but not to active caspase-8.

The band in the SDS-PAGE corresponding to p72 was excised, trypsin digested and subject to limited sequence analysis and to mass spectroscopy analysis. 7 peptides obtained by trypsin digestion were used to search a protein database deduced from nucleotide sequences (or ESTs). The protein sequence matched part of a predicted protein sequence of a human EST clone (SEQ:ID1) found in the gene bank (accession number gi/2988397/gbAAC08052.1/(AC004475) whose function was unknown.

### Example 8:

### Generation of the full-length cDNA encoding p72.

The full-length cDNA encoding p72 was generated as follows:

The EST from (Example 7) was used to screen a TIGR Human gene index and the THC report (THC510568 SEQ ID NO: 1) containing the consensus of all the ESTs, that fit this sequences was obtained.

A DNA clone encoding part of the predicted protein was purchased from Incyte Genomics (IMAGE #2964545). The clone lacked the nucleotide sequences encoding the first methionine and the 6 succeeding amino acids (i.e. 21 nucleotides). The mouse and human sequences of these proteins were found to be highly similar (about 90 % identity), thus the nucleotide sequences encoding the first methionine and the 6 succeeding amino acids of the mouse protein which were not missing in the mouse ESTs were compared to the working draft sequence of the human genome in order to complete the missing human sequence. A hit was obtained corresponding to the sequence of Homo sapiens chromosome 19, clone LLNLR-232E12. This clone confirmed the nucleotide sequence which encodes the missing 7 amino acids of p72. The full-length cDNA of p72 was obtained by two PCR rounds (Takara ExTaq, Takara, cat # R001A was used), which are schematically represented in Fig. 8.

In the first PCR the clone obtained from Incyte Genomics was used as the template with the forward primer: CTCAAGATGGACAACCGGGATGTTGCAGGAAAGG synthesized to contain 15 (underlined) out of the 21 missing nucleotides together with the existent sequence of p72 (Fig. 8 primer 2) and the reverse primer:
CCACTCGAGTCAGTAGTAAGGCCGTCTGGGATT containing the 3' region ending with the stop codon (Fig. 8 primer 3).

The second PCR comprises as the template the PCR product of the first PCR round and the forward primer: AATGGATCCATGAGTCTCAAGATGGACAACCGGGA containing the whole 21 missing nucleotides and 5 existent nucleotides (Fig. 7 primer 1) and the same reverse primer (Fig. 7 primer 3). The whole cDNA encoding p72 was recovered and sequenced (SEQ ID NO: 2) and the amino acid sequence was predicted from the nucleotide sequence (SEQ ID NO: 3).

Comparison of the sequence obtained in the THC report (THC510568 SEQ:ID 1) containing the consensus of all the ESTs and the protein predicted by the generated full length cDNA (SEQ:ID 3) in figure 13 shows the lacking first 7 amino acids in the ESTs the missing 25 amino acids sequence in the ESTs and inaccuracy of amino acid 397 (proline instead of leucine).

P72 protein was found to contain three conserved motifs (Figure 7): the C motif a coiled motif, two tandem located 'SURP' (also called 'SWAP'motifs, denoted as S Figure 7) (Denhez F and Lafyatis R 1994) close to the N terminus of the protein, and one C terminally located 'G-patch' (Figure 8 denoted as G) (Aravind L and Koonin EV 1999). Both the SURP and the G-patch motifs are believed to contribute to RNA-binding, suggesting that the target of p72 may be a RNA molecule.

### Example 9:

### Cleavage of p72 by caspase-8.

As shown in example 8, p72 is bound only to pro-caspase-8 and not to active caspase-8 as tested following one hour stimulation. Some pro-caspase-8 can be still detected after 20 minutes stimulation. To determine whether p72 can be co-precipitated with caspase-8 at shorter stimulation times, Bjab cells activated for only 20 minutes were lysed and immunoprecipitated with Mab 179. Following immunoprecipitation and elution, caspase-8 and bound proteins were resolved by SDS-PAGE and the proteins were detected by Silver staining. One band of 72.5 kDa (Fig. 9 lane 3) corresponding to p72 was immunoprecipitated in lysates from cells before stimulation while after 20 minutes stimulation a protein with a lower apparent molecular weight of about 68 kDa was detected (Fig. 9 lane 4). Both proteins, the 725 and 68 kDa, immunoprecipitated from Bjab cells, were subjected to mass spectroscopy analysis. After tripsynization, both proteins exhibited similar peptide profile except one clear difference, an additional peptide of sequence FRPNPLNNPR (residues 632-641) was present in the 72.5 kDa at the C-terminus protein but absent in the 68 kDa protein.

This result suggests that upon cell stimulation a fragment of about 45 kDa is removed from the C-terminus of p72, probably by activated caspase-8, resulting in a smaller protein with an apparent molecular weight of 68 kDa which is still bound to the remaining pro-caspase-8.

It is conceivable that residue D 600 located at the C-terminus of p72 (Fig. 8) could be a candidate residue for cleavage, because the putative fragments resulting from such a cleavage exhibit similar molecular weight as the p72 fragments detected *in-vivo* following 20 minutes stimulation.

In order to test whether, as suggested, p72 is a substrate of caspase-8 and D 600 is the target residue for cleavage, an in vitro transcripted-translated and radioisotope labelled (S³⁵) p72 (TnT system) was subjected to the action of recombinant active caspase-8. The protein encoded by the P72 cDNA was expressed in vitro in reticulocyte lysates in the presence of ³⁵S methionine using the TnT T7 Coupled Reticulocyte Lysate System, and subjected to cleavage by recombinant active caspase-8 (each Sub-unit 1 and 2 prepared separately in *E. coli* mixed and re-folded together in vitro). Briefly, p72 in-vitro synthesized ³⁵S labelled proteins were incubated for 30 min. in protease buffer (25 mM Hepes pH 75, 0.1 % CHAPS, 5mM EDTA and 2mM DTT) at 37°C in the presence of bacterially produced caspase-8. Proteins and their fragments were separated on SDS-PAGE and the results visualised by phospho-imaging. The results (Fig. 10) show that in the absence of caspase-8 only the 725 band corresponding to p72 (lane 1) is detected. This band disappears after addition of activated caspase-8 for 1 hour and a new smaller fragment corresponding to 68 kDa appears (lane 4). This result indicates that the protein encoded by the p72 cDNA used as substrate, is effectively cleaved by caspase-8. In addition, the TnT transcription translation system was used also to produce in vitro 2 different p72 mutants: p72 in which the residue D 600, suspected from the *in-vivo* experiments to be the target residue for caspase-8, was mutated to E (D600E), and a deleted p72 missing the residues down-stream D600 (i.e. the expressed protein will exhibit the 1-600 residues).

Cleavage of the above two p72 mutants was tested in the presence (Fig. 10 lanes 5 and 6, respectively) or in the absence (lanes 2 and 3, respectively) of activated recombinant caspase-8. As shown in Fig, 10 (lanes 3 and 6 respectively) the same protein profile of p72 D600E mutant is observed in the presence or absence of caspase-8, indicating that caspase-8 does not cleave the p72 D600E mutant. The p72 1-600 mutant co-migrates with the 68 kDa fragment produced after cleavage of the wild type p72 and is not further cleaved by addition of caspase- 8 (lanes 2 and 5). These results show that, upon activation, caspase-8 cleaves p72 at the D600 residue.

Studies carried out *in-vivo* suggest that cleavage of p72 occurs rapidly in cells, within 5-20 minutes after Fas ligand treatment (Fig. 11) and that the cleaved protein (or rather - its larger fragment) may remain associated with pro-caspase-8.

### Example 10 Western blot analysis for detection of caspase-8 immunoreactive serum.

A mixture of recombinant purified Sub-1 and Sub-2 was used for Western blot analysis of antibodies developed to synthetic peptides. Briefly a 12% SDS Poly Acryl amide gel was loaded with 100ng/lane of a mixture of Sub-1 and Sub-2 under reducing conditions (40mM DTT). One lane was loaded with Low Molecular Weight Markers (LMW). The proteins separated on the gels were transferred by electro elution to PVDF high bond-P (Amersham) membranes. The membranes were incubated in PBS containing 5% low-fat milk, 0.05% Tween 20, for 16 hr. The membranes were cut into strips and each strip was incubated for 1 hour at room temperature with the mouse antiserum (diluted 1/2000). Membrane strips were washed with PBS containing 0.05% Tween 20 (3x15 min) and incubated for one hour with the second antibody - goat anti-mouse conjugated to horseradish peroxidase (diluted 1:10.000, Jakson) for 1 hour at room temperature.

The strips were washed with PBS containing 0.1% Tween 20 (3x15 min). The positive bands were detected by enhanced chemiluminescence (ECL, Amersham).

For Western blots performed in example 5 antibodies specific to Sub-1 were used (Cell Signaling Technology Caspase-8 ICI2 Cat 9746).

### Example 11 ELISA for hybridoma clones screening

The direct ELISA for screening hybridoma producing specific antibody was performed as following: 96 wells plates were coated with 50µl/well of BSA-peptide (or BSA alone for control plates) at a concentration of 2.5µg/ml in binding solution (0.1 M Na₂HPO₄, pH 9) for 1 hour at 37°C or 16 hours at 4°C. Subsequently the plates were washed 3 times with PBS-T (PBS with 0.05% of Tween-20) and loaded with 200 µl/well of blocking solution (1% hemoglobin in PBS) for 1 hour at 37°C and washed 3 times with PBS. 50 µl of hybridoma culture supernatant or diluted standards (with PBS-T) were loaded per well and incubated for 1 hour at 37°C or 4 hours at 22 °C. After this incubation period the wells were washed 6 times with PBS-T. A second antibody, anti mouse antibody conjugated to HRP (Jackson 115-025-100) was diluted 1:5000 in PBS-T, incubated for 1 hour at 37°C and washed away by washing 6 times with PBS-T. The substrate for HRP was freshly prepared (2.2 ml of 0.2M Na₂HPO₄ pH 9.2,1.4 ml of 0.2 M citric acid, pH 4.35, 6.4 ml H₂O,10 mg ABTS and 1µl H₂O₂) and 50 µl/well were loaded and incubated at 22°C until color developed (about 5-80 minutes). The color reaction was stopped by adding 50 µl/well 0.2 M citric acid. The plates were read at 405 nm.

As a positive control antibody, positive mouse antisera diluted 1:1000 was used and as negative control media.

### Example 12 immunoprecipitation of caspase-8.

For every immunoprecipitation 10⁸ cells were used. Cells were collected and lysed by incubation in 1% NP-40 lysis buffer and complete protease inhibitor (complete protease inhibitor cocktail tablets from Roche Molecular Biochemicals) on 0°C for 40 min. The cell lysates were aliquoted in Eppendorf tubes, centrifuged at 14000 rpm for 10 minutes at 4°C and the supernatant collected in a new tube. The cell lysates were subjected to a pre-clearing step, intended to remove proteins that bind non-specifically to the protein-G-sepharose. For pre-clearing, cell lysate was pre-incubated with PBS pre-washed protein-G-sepharose (Pharmacia) and with mouse IgG for 2-3 hours at 4°C. Following this incubation the lysates were centrifuged in Eppendorf tubes for 14000rpm for 30 seconds, the proteinG-sepharose was discarded and the pre-cleared supernatant collected. Purified monoclonal antibody (or mouse IgG 1 kappa for negative controls) and PBS pre-washed protein-G-sepharose were mixed and incubated with the pre-cleared supernatant for 4-to16 hours at 4°C. Following this incubation period the unbound material denoted "depleted lysate" was collected by centrifugation (30 seconds at 14000 rpm) and the bound material was eluted by washing the sepharose beads 6 times with lysis buffer and by incubation with an "eluting solution" containing 0.2% NP-40 lysis buffer, protease inhibitors and 400µg/ml peptide used for immunization (300 µl eluting solution/100µl sepharose) for 2 hours at 22 °C. The tubes were spinned for 5 minutes at 5,000 rpm and the supernatant denoted "caspase-8 eluate" transferred into a new tube.

### REFERENCES

Adelman Jp et al. 1983 DNA 2:183. Ahmad M et al.Cancer Res 1997; 57(4):615-9.
Akagi Y et al.1997 Kidney Int. 51, p.1265-9.
Aravind L and Koonin EV 1999 TIBS 24: 342-344.
Artelt Pet al. 1988 Gene 68,213-9.
Barnikol-Watanabe S et al. Biol Chom Hoppe Seyler 1994;375(8):497-512.
Beidler Dret al. 1995. J Biol Chem 1995 Jul 14;270(28):16526-8.
Beutler and Cerami 1987 N Engl J Med 1987 Feb 12;316(7):379-85.
Bigda J et al. 1994 J Exp Med Aug 1;180(2):445-60
Boldin MP et al. 1995 J Biol Chem Apr 7;270(14):7795-8
Boldin MP et al.1995a Biol Chem 1995 Jan 6;270(1):387-91.
Boldin MP et al.1995b FEBS Lett 1995 Jun 19;367(1):39-44.
Boldin MP et al. 1996 Cell 1996 Jun 14;85(6):803-15.
Boone, E et al. J. Biol Chem. 275,37596-603.
Boulianne GL et al. 1984 Nature 1984 Dec 13-19;312(5995):643-6.
Brakebusch C et al. 1992. EMBO J 1992 Mar;11(3):943-50.
Brockhaus M et al. 1990 Proc Natl Acad Sci U S A 1990 Apr;87(8):3127-31.
Cabilly S et al. 1984 PNAS 81, 3273.
Cantor et al. 1993 PNAS 90 109-32.
Cao X et al.1998 J Immunol 161, 6238-44.
Chen CJ et al. 1992 Ann. NY Acad. Sci. 660,271-3.
Chinnaiyan AM et al. 1995 Cell May 19;81(4):505-12.
Chinnaiyan AM et al.1996 J Biol Chem Mar 1;271(9):4961-5.
Clements GB et al.1975 Int J Cancer 16:125-33.
Cohen OM 1997 Biochem J;326 (Pt 1).1-1 6.
Daniel R et al. 1998 J Biomed Sci. 5, 383-94.
Denhez F and Lafyatis R 1994 J Biol Chem 269: 16170-16179.
Dirks, Wirth and Hauser 1983 Gene 128, 247-249.
Duan H and Dixit VM. Nature 1997;385(6611):86-9.
Durfee T et al. Genes Dev 1993;7(4).SSS-69.
Edamatsu H, Kaziro Y, Itoh H.1997, Gene 187, 289-94.
Edinger AL et al.1998 Virology. 249, p. 367-78.
Enari M et al.1995. Nature May 4;375(6526):78-81
Enari M et al. 1998 Nature (London) 391,43-50.
Engelmann H et al.1990. J. Biol Chem Jan 25;265(3):1531-6.
Engelmann H et al.1990 J. Biol. Chem. 265, p. 14497-504.
Eshhar Z, 1985 in "Hybridoma technology in the bioscience and medicine", Edited by Timothy A Springer (Plenum Publishing Corporation, 1985; Chapter 1).
Everett RD et al. 1983 Nucleic Acids Res.112447-64.
Femandes-Alnemri T et al.1996 ProcNatiAcadSciUSA;93(15):7464-9
Fields S et al. 1989; Nature 340(6230):245-6.
Flanagan, 1998 Cancer Metastasis Rev 17,169-76.
Furth PA et al. 1994 ProcNatiAcadSciUSA 91, 9302-6.
Gerster M et al. 1998 Anal Biochem. 262,177-84.
Graham A et al.1991, Biochem Biophys Res Commun 177, 8-16.
Grau GE 1989 Schweiz Med Wochenschr Dec 9;119(49):1756-61.
Griscell F et al. 1998, Hum Gene Ther. 9,1919-28.
Guang-Lin M et al. 1998 Transplant Proc 30, 2923-4.
Guinot and Temsamani, 1998 Pathol Biol (Paris) 46, p. 347-54.
Haendler B et al. E. EMBO J 1987;6(4):947-50.
Hemmi S et al. 1998 Hum Gene Ther 9, p. 2363-73.
Henkart, PA 1996. Immunity Mar;4(3):195-201
Hohmann HP et al. 1989. J Biol Chem Sep 5;264(25):14927-34
Hsu H et al. 1996. Cell Jan 26;84(2):299-308
Huang MT and Gorman CM. 1990 Nucleic Acids Res.18, 937-47.
Ihle JN Cell 1996;84(3).331-4.
Itoh N et al.1991. Cell Jul 26;66(2):233-43.
Itoh and Nagata 1993 J. Biol Chem May 25;268(15):10932-7.
Janmey, P. A., and Stossel, T.P. 1987 Nature (London) 352,362-364.
Joseph and Burke, 1993J. Biol. Chem. 268, 24515.
Kamine J et al. Feb 15;216(2):357-66.
King P and Goodboum S. J Biol Chem 1998;273(15) 8699-704
Kischkel FC et al. EMBO J 1995; 14(22);5579-88.
Kohler and Milstein, 1975 Nature 256,495-497.
Kondo S et al.1998 Oncogene 17, 2585-91.
Kothakota, S et al. 1997 Science 278, 294-298.
Kozak, M, 1984 Nucleic Acids Res. 12 p. 857-72.
Kumar, 1995 Trends Biochem Sci May;20(5):198-202.
Kumar, 1997 Science 278(5343):1630-2.
Kunstle, G et al.1997 Immunol Lett. 55, 5-10.
Kurth, M., and Bryan, J.1984 J. Biol. Chem. 259,10895-10903.
Loetscher H et al. 1990 J Biol Chem 1990 Nov 25;265(33):20131-8.
MacFarlane M et al. JBiol Chem 1997;272(41):25417-20.
Meinkoth et al. (1984).
Meiri N et al. 1998 Proc Natl Acad Sci U S A 95 15037-15042.
Mittl PR et al JBiol Chem 1997;272(10):6539-47
Miura K et al. Biochem Blophys Res Commun 1992; 1 87(1),3 75 ―80.
Morrison et al.,1984 PNAS 81,6851.
Muranishi et al., 1991Pharm. Research 8, 649.
Muzio et al.1996. Cell Jun 14;85(6):817-27.
Muzio M et al JBiol Chem 1998.,273(5):2926-30.
Nagata and Goldstein 1995.
Nagata S et al. Cell 1997;88(3):355-65.
Nakashima A et al. JBiol Chem 1997;272(14):9567-72.
Narumi et al.,1998 Am J Respir Cell Mol Biol 19, 936-941.
Nicholson D.W. and Thomberry, N.A. 1997 Trends Biochem. Sci. 22,299-306.
Nishida et al.,1998 Spine 23, 2437-42.
Nophar et al. 1990.
Ohtsu, M et al. 1997 EMBO J.16, 4650-4656.
Pederson et al,1998 J Gastrointest Surg 2, 283-91.
Quelle FW et al. J Biol Chem 1995;270(35):20775-80.
Rotonda J et al Nat Struct Siol 1996;3(7):619-25.
Ruzicka et al. 1993 Science 260:487.
Sakahira, H., et al. (1998) Nature (London) 391, 96-99.
Salvesen, G. S., and Dixit, V. M. (1997) Cell 91,443-446.
Sambrook et al.,1989.
Sano et al ., (1991) Biotechniques 9:1378.
Sano et al., (1992) Science 258:120.
Schall et al. 1990
Schek et al., Mol Cell Biol., p. 5386-93, 1992.
Schwarzenberger et al., 1998 J Immunol 161, 6383-9.
Shevchenko A, et al. 1997 Rapid Commun Mass Spectrom. 11, p.1015-24.
Shimayama et al., 1993 Nucleic Acids Symp Ser 29, 177.
Shoji et al., 1998 J Drug Target 5 261-73.
Shore et al.,1993 Oncogene 8, 3183.
Smith et al. 1990 J Infect Dis Dec;162(6):1349-53
Soukchareun et al., 1998 Bioconjug Chem 9 466-75.
Srinivasula SM et al.1996 ProcNatlAcadSciUSA;93(25):14486-91
Srinivasula SM et al. JBiot Chem 1998;273(17) 10107-11.
Stanger et al.1995 Cell May 19;81(4):513-23
Steinitz M and Klein G.Proc Natl Acad Sci U S A 1975 Sep; 72(9): 3518-20.
Stix 1998 Sci Am. 279 46-50.
Surinya et al. J Biol Chem. 273, p.16798-809,1998
Tartaglia et al.1993 Cell Sep 10;74(5):845-53.
Teoh et al.,1998 Blood 92, 4591-4601.
Tewari et a1.1995 Cell Jun 2;81(5):801-9.
Thomsen, et al.1984 PNAS 81 659-63.
Thornberry NA et al. JBiol Chem 1997;272(29):17907-1 1
Tirode F et al. J Biol Chem 1997;272(3 7):22995-9.
Tokushige, et al.,1997 J Virol Methods. 64 p. 73-80.
Tracey et al.1986 Annu Rev Med 45:491-503 Vandenabeele et al. 1995 J Immunol Mar 15;154(6):2904-13
Vercammen, D., et al. 1998 J Exp Med. 4,1477-85.
Vielkind and Swierenga 1989 Histochemistry ;91(1):81-8.
Villa, Pet al. (1997) Trends Biochem. Sci. 22, 388-393.
Vincenz C and Dixit VM 1997. J Biol Chem. Mar 7;272(10):6578-83
Wahl et al., 1983 J. Nucl. Med. 24:316-325.
Wallach, D et al. 1999 Annu Rev Immunol. 17,331-67.
Wang, J 1998 Controlled Release 53, 39-48.
Weaver et al. 2000 Hybridoma Apr ; 19 (2) :167-9.
Xue et al. 1995 Nature Sep 21;377(654b):248-51
Yamamoto et al.1980. Cell 22, 787-97.
Yamamoto T et al. JBiol Chem 1997;272(49):30595-8
Yang X et al. Mol Cell 1998; 1(2).319-25.
Yeh et al. 1998 J Bone Miner Res. 13. p.1870-9.
Yin, H.L. and Stossel, T.P. (1979) Nature (London) 281, 583-586.
Yin, H.L. and Stossel, T.P., (1980) J. Biol. Chem. 255, 9490-9493.
Zacharia et al.,1991 Eur. J. Pharmacol. 203, 353.
Zhang et al.1998 Clin Cancer Res 4, 2669-76.
Zhao and Pick, 1993 Nature 365, 448.

## Claims

1. An antibody obtained by immunization of an animal with a peptide from the C-terminal end of the caspase-8 Sub-1 unit, and fragments thereof, wherein the peptide used for immunization has the amino acid sequence CQGDNYQKGIPVETD (SEQ ID No: 4), capable of co-immunoprecipitating said caspase (both active caspase-8 and pro-caspase-8) together with the pro-caspase-8 associated protein p72 (SEQ ID No: 3), and of releasing the caspase and associated protein efficiently from the immune complex upon elution using the peptide according to SEQ ID No: 4.

2. An antibody according to claim 1, being a polyclonal antibody or fragment thereof.

3. An antibody according to claim 1, being a monoclonal antibody or fragment thereof.

4. An antibody according to claim 1, being a chimeric antibody or fragment thereof.

5. An antibody according to claim 1, being a fully humanized antibody or fragment thereof.

6. An antibody according to claim 1, being an anti-anti-ld antibody or fragment thereof.

7. An antibody according to any one of claims 1-6, wherein the peptide used for immunization is coupled to KLH.

8. An antibody according to any one of claims 1-7, being of the immunoglobulin isotype IgG₁.

9. An antibody according to claim 8, wherein the antibody triggers processing of caspase-8.

10. The use of an antibody according to any one of claims 1-9, for the development of an ELISA assay.

11. A method for preparing an antibody according to claims 1 or 2, comprising immunizing an animal with a peptide from the C-terminal end of Sub-1 of caspase-8 wherein the peptide used for immunization has the amino acid sequence CQGDNYQKGIPVETD (SEQ ID No: 4).

12. A method according to claim 11, wherein the antibody is monoclonal.

13. A method according to any one of claims 11-12, wherein the immunogen is linked to a carrier.

14. A method according to claim 13, wherein the carrier is KLH.

15. A method for the purification of a caspase and caspase-bound protein, which comprises contacting a sample containing a caspase and the caspase-bound protein with an antibody according to any one of claims 1-9, co-immunoprecipitating the caspase and caspase-bound protein, washing the immune complex produced, and recovering the caspase and the caspase-bound protein from the immune complex using CQGDNYQKGIPVETD (SEQ ID No: 4) as a competing peptide.

16. A method according to claim 15, wherein the sample is selected from body fluids, cell extracts and DNA expression libraries.

17. A method according to any one of claims 15 and 16, wherein the competing peptide comprises the amino acid sequence CQGDNYQKGIPVETD (SEQ ID No: 4).

18. A method according to any one of claims 15-17 wherein the cells in the sample were stimulated prior to extraction.

19. A method according to any one of claims 15-18, wherein the caspase is caspase-8.

20. The method according to any one of claims 15-19, wherein additionally an antibody obtainable by immunization of an animal with the peptide according to SEQ ID No: 6 is used.

21. The use of epitope 179 (SEQ ID No: 4) for obtaining an antibody according to any one of claims 1-6, comprising immunization of an animal with such an epitope.

## Patentansprüche

1. Antikörper, erhalten durch Immunisierung eines Lebewesens mit einem Peptid aus dem C-terminalen Ende der Caspase-8 Sub-1-Einheit, und Fragmente davon, worin das Peptid, das zur Immunisierung verwendet wird, die Aminosäuresequenz CQGDNYQKGIPVETD (SEQ ID Nr.: 4) aufweist, der in der Lage ist zum Copräzipitieren der Caspase (sowohl aktive Caspase-8 als auch pro-Caspase-8) zusammen mit dem pro-Caspase-8-assoziierten Protein p72 (SEQ ID Nr.:3), und zum wirkungsvollen Freisetzen von Caspase und assoziiertem Protein von dem Immunkomplex bei Elution unter Verwendung des Peptids entsprechend SEQ ID Nr.: 4.

2. Antikörper nach Anspruch 1, der ein polyklonaler Antikörper oder ein Fragment davon ist.

3. Antikörper nach Anspruch 1, der ein monoklonaler Antikörper oder ein Fragment davon ist.

4. Antikörper nach Anspruch 1, der ein chimärer Antikörper oder ein Fragment davon ist.

5. Antikörper nach Anspruch 1, der ein vollständig humanisierter Antikörper oder ein Fragment davon ist.

6. Antikörper nach Anspruch 1, der ein anti-anti-Id-Antikörper oder ein Fragment davon ist.

7. Antikörper nach einem der Ansprüche 1-6, worin das zur Immunisierung verwendete Peptid an KLH gekoppelt ist.

8. Antikörper nach einem der Ansprüche 1-7, der aus dem Immunglobulin-Isotyp IgG₁ ist.

9. Antikörper nach Anspruch 8, worin der Antikörper das Prozessieren von Caspase-8 auslöst.

10. Verwendung eines Antikörpers nach einem der Ansprüche 1-9 zur Entwicklung eines ELISA-Assays.

11. Verfahren zur Herstellung eines Antikörpers nach Anspruch 1 oder 2, umfassend Immunisieren eines Lebewesens mit einem Peptid von dem C-terminalen Ende von Sub-1 von Caspase-8, worin das Peptid, das zur Immunisierung verwendet wird, die Aminosäuresequenz CQGDNYQKGIPVETD (SEQ ID Nr.: 4) aufweist.

12. Verfahren nach Anspruch 11, worin der Antikörper monoklonal ist.

13. Verfahren nach einem der Ansprüche 11-12, worin das Immunogen an einen Träger gebunden ist.

14. Verfahren nach Anspruch 13, worin der Träger KLH ist.

15. Verfahren zur Reinigung einer Caspase und von Caspase-gebundenem Protein, umfassend das Inkontaktbringen einer Probe, die eine Caspase und das Caspase-gebundenes Protein enthält, mit einem Antikörper nach einem der Ansprüche 1-9, Co-Immunpräzipitieren der Caspase und des Caspase-gebundenen Proteins, Waschen des erzeugten Immunkomplexes und Gewinnen der Caspase und des Caspase-gebundenen Proteins aus dem Immunkomplex, unter Verwendung von CQGDNYQKGIPVETD (SEQ ID Nr.: 4) als ein kompetetierendes Peptid.

16. Verfahren nach Anspruch 15, worin die Probe ausgewählt wird aus Körperfluiden, Zellextrakten und DNA-Expressionsbibliotheken.

17. Verfahren nach einem der Ansprüche 15 und 16, worin das kompetetierende Peptid die Aminosäuresequenz CQGDNYQKGIPVETD (SEQ ID Nr.: 4) umfasst.

18. Verfahren nach einem der Ansprüche 15-17, worin die Zellen in der Probe vor Extraktion stimuliert wurden.

19. Verfahren nach einem der Ansprüche 15-18, worin die Caspase Caspase-8 ist.

20. Verfahren nach einem der Ansprüche 15-19, worin zusätzlich ein Antikörper, der erhältlich ist durch Immunisierung eines Lebewesens mit dem Peptid entsprechend SEQ ID Nr.: 6, verwendet wird.

21. Verwendung von Epitop 179 (SEQ ID Nr.:4) zum Erhalten eines Antikörpers nach einem der Ansprüche 1-6, umfassen das Immunisieren eines Lebewesens mit einem solchen Epitop.

## Revendications

1. Anticorps obtenu par immunisation d'un animal avec un peptide provenant de l'extrémité C-terminale de la sous-unité 1 de caspase-8, et ses fragments, dans lequel le peptide utilisé pour l'immunisation a la séquence d'acides aminés CQGDNYQKGIPVETD (SEQ ID N° 4), capable de co-immunoprécipiter ladite caspase (tant la caspase-8 active que la pro-caspase-8) conjointement avec la protéine p72 associée à la pro-caspase-8 (SEQ ID N° 3) et de libérer la caspase et la protéine associée efficacement à partir du complexe immun par élution utilisant le peptide selon la SEQ ID N° 4.

2. Anticorps selon la revendication 1, qui est un anticorps polyclonal ou un fragment de celui-ci.

3. Anticorps selon la revendication 1, qui est un anticorps monoclonal ou un fragment de celui-ci.

4. Anticorps selon la revendication 1, qui est un anticorps chimère ou un fragment de celui-ci.

5. Anticorps selon la revendication 1, qui est un anticorps complètement humanisé ou un fragment de celui-ci.

6. Anticorps selon la revendication 1, qui est un anticorps anti-anti-Id ou un fragment de celui-ci.

7. Anticorps selon l'une quelconque des revendications 1 à 6, dans lequel le peptide utilisé pour l'immunisation est couplé à la KLH.

8. Anticorps selon l'une quelconque des revendications 1 à 7, qui est de l'isotype d'immunoglobuline IgG₁.

9. Anticorps selon la revendication 8, dans lequel l'anticorps déclenche la transformation de caspase-8.

10. Utilisation d'un anticorps selon l'une quelconque des revendications 1 à 9, pour le développement d'un dosage ELISA.

11. Procédé pour préparer un anticorps selon la revendication 1 ou 2, comprenant l'immunisation d'un animal avec un peptide provenant de l'extrémité C-terminale de la sous-unité 1 de caspase-8, dans lequel le peptide utilisé pour l'immunisation a la séquence d'acides aminés CQGDNYQKGIPVETD (SEQ ID N° 4).

12. Procédé selon la revendication 11, dans lequel l'anticorps est monoclonal.

13. Procédé selon l'une quelconque des revendications 11 et 12, dans lequel l'immunogène est lié à un support.

14. Procédé selon la revendication 13, dans lequel le support est la KLH.

15. Procédé pour la purification d'une caspase et d'une protéine liée à la caspase, qui comprend la mise en contact d'un échantillon contenant une caspase et la protéine liée à la caspase avec un anticorps selon l'une quelconque des revendications 1 à 9, la co-immunoprécipitation de la caspase et de la protéine liée à la caspase, le lavage du complexe immun produit, et la récupération de la caspase et de la protéine liée à la caspase à partir du complexe immun par utilisation de CQGDNYQKGIPVETD (SEQ ID N° 4) en tant que peptide compétiteur.

16. Procédé selon la revendication 15, dans lequel l'échantillon est choisi parmi les fluides corporels, les extraits cellulaires et les bibliothèques d'expression d'ADN.

17. Procédé selon l'une quelconque des revendications 15 et 16, dans lequel le peptide compétiteur comprend la séquence d'acides aminés CQGDNYQKGIPVETD (SEQ ID N° 4).

18. Procédé selon l'une quelconque des revendications 15 à 17, dans lequel les cellules dans l'échantillon ont été stimulées avant extraction.

19. Procédé selon l'une quelconque des revendications 15 à 18, dans lequel la caspase est la caspase-8.

20. Procédé selon l'une quelconque des revendications 15 à 19, dans lequel, de plus, on utilise un anticorps pouvant être obtenu par immunisation d'un animal avec le peptide selon la SEQ ID N° 6.

21. Utilisation de l'épitope 179 (SEQ ID N° 4) pour l'obtention d'un anticorps selon l'une quelconque des revendications 1 à 6, comprenant l'immunisation d'un animal avec un tel épitope.


### SEQUENCE LISTING

<110> Yeda Research and Development Co. Ltd
   wallach, David
   Goncharov, Tanya
   Kolumam, Ganesh
<120> Antibodies against Caspase-8 , their preparation and use
<130> 484
<150> 145279
   <151> 2001-04-09
<160> 6
<170> PatentIn version 3.1
<210> 1
   <211> 616
   <212> PRT
   <213> Homo sapiens
<400> 1
   Ala Arg Gly Arg Asp Val Ala Gly Lys Ala Asn Arg Trp Phe Gly Val
   1               5                   10                  15
   Ala Pro Pro Lys Ser Gly Lys Met Asn Met Asn Ile Leu His Gln Glu
               20                  25                  30
   Glu Leu Ile Ala Gln Lys Lys Arg Glu Ile Glu Ala Lys Met Glu Gln
           35                  40                  45
   Lys Ala Lys Gln Asn Gln Val Ala Ser Pro Gln Pro Pro His Pro Gly
       50                  55                  60
   Glu Ile Thr Asn Ala His Asn Ser Ser Cys Ile Ser Asn Lys Phe Ala
   65                  70                  75                  80
   Asn Asp Gly Ser Phe Leu Gln Gln Phe Leu Asn Ala Gly Lys Arg Ser
   85 90 95
   Leu Leu Ile Ser Arg Arg Thr Gly Leu Gly Leu Ala Ser Leu Pro Gly
   100 105 110
   Pro Val Lys Ser Tyr Ser His Ala Lys Gln Leu Pro Val Ala His Arg
   115 120 125
   Pro Ser Val Phe Gln Ser Pro Asp Glu Asp Glu Glu Glu Asp Tyr Glu
   130 135 140
   Gln Trp Leu Glu Ile Lys Val Ser Pro Pro Glu Gly Ala Glu Thr Arg
   145 150 155 160
   Lys Val Ile Glu Lys Leu Ala Arg Phe Val Ala Glu Gly Gly Pro Glu
   165 170 175
   Leu Glu Lys Val Ala Met Glu Asp Tyr Lys Asp Asn Pro Ala Phe Ala
   180 185 190
   Phe Leu His Asp Lys Asn Ser Arg Glu Phe Leu Tyr Tyr Arg Lys Lys
   195 200 205
   Val Ala Glu Ile Arg Lys Glu Ala Gln Lys Ser Gln Ala Ala Ser Gln
   210 215 220
   Lys Val Ser Pro Pro Glu Asp Glu Glu Val Lys Asn Leu Ala Glu Lys
   225 230 235 240
   Leu Ala Arg Phe Ile Ala Asp Gly Gly Pro Glu Val Glu Thr Ile Ala
   245 250 255
   Leu Gln Asn Asn Arg Glu Asn Gln Ala Phe Ser Phe Leu Tyr Glu Pro
   260 265 270
   Asn Ser Gln Gly Tyr Lys Tyr Tyr Arg Gln Lys Leu Glu Glu Phe Arg
   275 280 285
   Lys Ala Lys Ala Ser Ser Thr Gly Ser Phe Thr Ala Pro Asp Pro Gly
   290 295 300
   Leu Lys Arg Lys Ser Pro Pro Glu Ala Leu Ser Gly Ser Leu Pro Pro
   305 310 315 320
   Ala Thr Thr Cys Pro Ala Ser Ser Thr Pro Ala Pro Thr Ile Ile Pro
   325 330 335
   Ala Pro Ala Ala Pro Gly Lys Pro Ala Ser Ala Ala Thr Val Lys Arg
   340 345 350
   Lys Arg Lys Ser Arg Trp Gly Pro Glu Glu Asp Lys Val Glu Leu Pro
           355                 360                 365
   Pro Ala Glu Leu Val Gln Arg Asp Val Asp Ala Ser Pro Ser Pro Leu
       370                 375                 380
   Ser Val Gln Asp Leu Lys Gly Leu Gly Tyr Glu Lys Gly Lys Pro Val
   385                 390                 395                 400
   Gly Leu Val Gly Val Thr Glu Leu Ser Asp Ala Gln Lys Lys Gln Leu
                   405                 410                 415
   Lys Glu Gln Gln Glu Met Gln Gln Met Tyr Asp Met Ile Met Gln His
               420                 425                 430
   Lys Arg Ala Met Gln Asp Met Gln Leu Leu Trp Glu Lys Ala Val Gln
           435                 440                 445
   Gln His Gln His Gly Tyr Asp Ser Asp Glu Glu Val Asp Ser Glu Leu
       450                 455                 460
   Gly Thr Trp Glu His Gln Leu Arg Arg Met Glu Met Asp Lys Thr Arg
   465                 470                 475                 480
   Glu Trp Ala Glu Gln Leu Thr Lys Met Gly Arg Gly Lys His Phe Ile
                   485                 490                 495
   Gly Asp Phe Leu Pro Pro Asp Glu Leu Glu Lys Phe Met Glu Thr Phe
               500                 505                 510
   Lys Ala Leu Lys Glu Gly Arg Glu Pro Asp Tyr Ser Glu Tyr Lys Glu
           515                 520                 525
   Phe Lys Leu Thr Val Glu Asn Ile Gly Tyr Gln Met Leu Met Lys Met
       530                 535                 540
   Gly Trp Lys Glu Gly Glu Gly Leu Gly Ser Glu Gly Gln Gly Ile Lys
   545                 550                 555                 560
   Asn Pro Val Asn Lys Gly Thr Thr Thr Val Asp Gly Ala Gly Phe Gly
                   565                 570                 575
   Ile Asp Arg Pro Ala Glu Leu Ser Lys Glu Asp Asp Glu Tyr Glu Ala
               580                 585                 590
   Phe Arg Lys Arg Met Met Leu Ala Tyr Arg Phe Arg Pro Asn Pro Leu
           595                 600                 605
   Asn Asn Pro Arg Arg Pro Tyr Tyr
       610                 615
<210> 2
   <211> 1938
   <212> DNA
   <213> Homo sapiens
<400> 2
   atgagtctca agatggacaa ccgggatgtt gcaggaaagg ctaaccggtg gtttggggtt     60
   gctcccccta aatctggaaa aatgaacatg aacatccttc accaggaaga gctcatcgct    120
   cagaagaaac gggaaattga agccaaaatg gaacagaaag ccaagcagaa tcaggtggcc    180
   agccctcagc ccccacatcc tggcgaaatc acaaatgcac acaactcttc ctgcatttcc    240
   aacaagtttg ccaacgatgg tagcttcttg cagcagtttc tgaagttgca gaaggcacag    300
   accagcacag acgccccgac cagtgcgccc agcgcccctc ccagcacacc cacccccagc    360
   gctgggaaga ggtccctgct catcagcagg cggacaggcc tggggctggc cagcctgccg    420
   ggccctgtga agagctactc ccacgccaag cagctgcccg tggcgcaccg cccgagtgtc    480
   ttccagtccc ctgacgagga cgaggaggag gactatgagc agtggctgga gatcaaagtt    540
   tcacccccag agggagccga gactcggaaa gtgatagaga aattggcccg ctttgtggca    600
   gaaggaggcc ccgagttaga aaaagtagct atggaggact acaaggataa cccagcattt    660
   gcatttttgc acgataagaa tagcagggaa ttcctctact acaggaagaa ggtggctgag    720
   ataagaaagg aagcacagaa gtcgcaggca gcctctcaga aagtttcacc cccagaggac    780
   gaagaggtca agaaccttgc agaaaagttg gccaggttca tagcggacgg gggtcccgag    840
   gtggaaacca ttgccctcca gaacaaccgt gagaaccagg cattcagctt tctgtatgag    900
   cccaatagcc aagggtacaa gtactaccga cagaagctgg aggagttccg gaaagccaag    960
   gccagctcca caggcagctt cacagcacct gatcccggcc tgaagcgcaa gtcccctcct   1020
   gaggccctgt cagggtcctt acccccagcc accacctgcc ccgcctcgtc cacgcctgcg   1080
   cccactatca tccctgctcc agctgccccc gggaagccag cctccgcagc caccgtgaag   1140
   aggaagcgga agagccggtg ggggcctgaa gaggataagg tagagctcct acctgctgaa   1200
   ctggtgcaga gggacgtgga tgcctctccc tcgcctctgt cagttcagga cctcaagggg   1260
   ctcggctatg agaaggggaa gcctgtgggt ctagtgggcg tcacagagct ttcagacgcc   1320
   cagaagaagc agctgaagga gcagcaggag atgcagcaga tgtacgacat gatcatgcag   1380
   cacaagcggg ccatgcagga catgcagctg ctgtgggaga aggcagtcca acagcaccag   1440
   cacggctatg acagtgatga ggaggtggac agcgagctgg gcacctggga gcaccagctg   1500
   cggcgcatgg agatggataa gaccagggaa tgggccgagc agctgacaaa gatgggccgg   1560
   ggcaagcact tcatcggaga cttcctgcct ccagacgagc tggaaaagtt tatggagacc   1620
   ttcaaggccc tgaaggaggg ccgtgagcct gactactcag agtacaagga gttcaagctg   1680
   actgtggaga acatcggcta ccagatgctg atgaagatgg gctggaagga gggcgagggg   1740
   ctgggctcag agggccaggg catcaagaac ccagtgaaca agggcaccac cacagtggac   1800
   ggcgctggct tcggcattga ccggccggcg gagctctcca aggaggacga cgagtatgag   1860
   gcgttccgca agaggatgat gctggcctac cgcttccggc ccaaccccct gaacaatccc   1920
   agacggcctt actactga                                                  1938
<210> 3
   <211> 645
   <212> PRT
   <213> Homo sapiens
<400> 3
   Met Ser Leu Lys Met Asp Asn Arg Asp Val Ala Gly Lys Ala Asn Arg
   1               5                   10                  15
   Trp Phe Gly Val Ala Pro Pro Lys Ser Gly Lys Met Asn Met Asn Ile
               20                  25                  30
   Leu His Gln Glu Glu Leu Ile Ala Gln Lys Lys Arg Glu Ile Glu Ala
           35                  40                  45
   Lys Met Glu Gln Lys Ala Lys Gln Asn Gln Val Ala Ser Pro Gln Pro
       50                  55                  60
   Pro His Pro Gly Glu Ile Thr Asn Ala His Asn Ser Ser Cys Ile Ser
   65                  70                  75                  80
   Asn Lys Phe Ala Asn Asp Gly Ser Phe Leu Gln Gln Phe Leu Lys Leu
                   85                  90                  95
   Gln Lys Ala Gln Thr Ser Thr Asp Ala Pro Thr Ser Ala Pro Ser Ala
               100                 105                 110
   Pro Pro Ser Thr Pro Thr Pro Ser Ala Gly Lys Arg Ser Leu Leu Ile
           115                 120                 125
   Ser Arg Arg Thr Gly Leu Gly Leu Ala Ser Leu Pro Gly Pro Val Lys
       130                 135                 140
   Ser Tyr Ser His Ala Lys Gln Leu Pro Val Ala His Arg Pro Ser Val
   145                 150                 155                 160
   Phe Gln Ser Pro Asp Glu Asp Glu Glu Glu Asp Tyr Glu Gln Trp Leu
                   165                 170                 175
   Glu Ile Lys Val Ser Pro Pro Glu Gly Ala Glu Thr Arg Lys Val Ile
               180                 185                 190
   Glu Lys Leu Ala Arg Phe Val Ala Glu Gly Gly Pro Glu Leu Glu Lys
   195 200 205
   Val Ala Met Glu Asp Tyr Lys Asp Asn Pro Ala Phe Ala Phe Leu His
   210 215 220
   Asp Lys Asn Ser Arg Glu Phe Leu Tyr Tyr Arg Lys Lys Val Ala Glu
   225 230 235 240
   Ile Arg Lys Glu Ala Gln Lys Ser Gln Ala Ala Ser Gln Lys Val Ser
   245 250 255
   Pro Pro Glu Asp Glu Glu Val Lys Asn Leu Ala Glu Lys Leu Ala Arg
   260 265 270
   Phe Ile Ala Asp Gly Gly Pro Glu Val Glu Thr Ile Ala Leu Gln Asn
   275 280 285
   Asn Arg Glu Asn Gln Ala Phe Ser Phe Leu Tyr Glu Pro Asn Ser Gln
   290 295 300
   Gly Tyr Lys Tyr Tyr Arg Gln Lys Leu Glu Glu Phe Arg Lys Ala Lys
   305 310 315 320
   Ala Ser Ser Thr Gly Ser Phe Thr Ala Pro Asp Pro Gly Leu Lys Arg
   325 330 335
   Lys Ser Pro Pro Glu Ala Leu Ser Gly Ser Leu Pro Pro Ala Thr Thr
   340 345 350
   Cys Pro Ala Ser Ser Thr Pro Ala Pro Thr Ile Ile Pro Ala Pro Ala
   355 360 365
   Ala Pro Gly Lys Pro Ala Ser Ala Ala Thr Val Lys Arg Lys Arg Lys
   370 375 380
   Ser Arg Trp Gly Pro Glu Glu Asp Lys Val Glu Leu Leu Pro Ala Glu
   385 390 395 400
   Leu Val Gln Arg Asp Val Asp Ala Ser Pro Ser Pro Leu Ser Val Gln
   405 410 415
   Asp Leu Lys Gly Leu Gly Tyr Glu Lys Gly Lys Pro Val Gly Leu Val
   420 425 430
   Gly Val Thr Glu Leu Ser Asp Ala Gln Lys Lys Gln Leu Lys Glu Gln
   435 440 445
   Gln Glu Met Gln Gln Met Tyr Asp Met Ile Met Gln His Lys Arg Ala
   450 455 460
   Met Gln Asp Met Gln Leu Leu Trp Glu Lys Ala Val Gln Gln His Gln
   465 470 475 480
   His Gly Tyr Asp Ser Asp Glu Glu Val Asp Ser Glu Leu Gly Thr Trp
   485 490 495
   Glu His Gln Leu Arg Arg Met Glu Met Asp Lys Thr Arg Glu Trp Ala
   500 505 510
   Glu Gln Leu Thr Lys Met Gly Arg Gly Lys His Phe Ile Gly Asp Phe
   515 520 525
   Leu Pro Pro Asp Glu Leu Glu Lys Phe Met Glu Thr Phe Lys Ala Leu
   530 535 540
   Lys Glu Gly Arg Glu Pro Asp Tyr Ser Glu Tyr Lys Glu Phe Lys Leu
   545 550 555 560
   Thr Val Glu Asn Ile Gly Tyr Gln Met Leu Met Lys Met Gly Trp Lys
   565 570 575
   Glu Gly Glu Gly Leu Gly Ser Glu Gly Gln Gly Ile Lys Asn Pro Val
   580 585 590
   Asn Lys Gly Thr Thr Thr Val Asp Gly Ala Gly Phe Gly Ile Asp Arg
   595 600 605
   Pro Ala Glu Leu Ser Lys Glu Asp Asp Glu Tyr Glu Ala Phe Arg Lys
   610 615 620
   Arg Met Met Leu Ala Tyr Arg Phe Arg Pro Asn Pro Leu Asn Asn Pro
   625 630 635 640
   Arg Arg Pro Tyr Tyr
   645
<210> 4
   <211> 15
   <212> PRT
   <213> artificial sequence
<220>
   <223> Peptide 179
<400> 4
   Cys Gln Gly Asp Asn Tyr Gln Lys Gly Ile Pro Val Glu Thr Asp
   1 5 10 15
<210> 5
   <211> 15
   <212> PRT
   <213> artificial sequence
<220>
   <223> Peptide 182
<400> 5
   Leu Ser Ser Pro Gln Thr Arg Tyr Ile Pro Asp Glu Ala Asp Cys
   1               5                   10                  15
<210> 6
   <211> 18
   <212> PRT
   <213> artificial sequence
<220>
   <223> Peptide 183
<400> 6
   Ser Glu Ser Gln Thr Leu Asp Lys Val Tyr Gln Met Lys Ser Lys Pro
   1               5                   10                  15
   Arg Cys